(19) Europäisches Patentamt
European Patent Office
Office européen des brevets

(11) **EP 4 055 389 B1**

(12) **EUROPEAN PATENT SPECIFICATION**

(45) Date of publication and mention
of the grant of the patent:
**11.09.2024 Bulletin 2024/37**

(21) Application number: **20800183.4**

(22) Date of filing: **06.11.2020**

(51) International Patent Classification (IPC):
*G01N 33/68* (2006.01)   *A61K 38/00* (2006.01)
*A61K 45/00* (2006.01)

(52) Cooperative Patent Classification (CPC):
**G01N 33/68; A61K 35/741; A61K 38/04;
A61K 38/164**

(86) International application number:
**PCT/EP2020/081256**

(87) International publication number:
**WO 2021/089764 (14.05.2021 Gazette 2021/19)**

(54) **DIAGNOSTICS AND USE OF QUORUM SENSING MOLECULES IN MUSCLE WASTING**

DIAGNOSE UND VERWENDUNG VON QUORUM-SENSORMOLEKÜLEN BEI MUSKELSCHWUND

DIAGNOSTICS ET UTILISATION DE MOLÉCULES DE DÉTECTION DE QUORUM DANS L'AMYOTROPHIE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **07.11.2019 EP 19207716**

(43) Date of publication of application:
**14.09.2022 Bulletin 2022/37**

(73) Proprietors:
• **Universiteit Gent**
**9000 Gent (BE)**
• **VIB VZW**
**9052 Gent (BE)**

(72) Inventors:
• **DE SPIEGELEER, Bart**
**9000 Gent (BE)**
• **DE SPIEGELEER, Anton**
**9000 Gent (BE)**
• **WYNENDAELE, Evelien**
**9550 Herzele (BE)**
• **ELEWAUT, Dirk**
**9070 Heusden (BE)**
• **VAN DEN NOORTGATE, Nele**
**9000 Gent (BE)**

(56) References cited:
**WO-A1-2012/159216    US-A1- 2015 335 688**

• **ARUNAVA BANDYOPADHAYA ET AL:
"Bacterial-excreted small volatile molecule
2-aminoacetophenone induces oxidative stress
and apoptosis in murine skeletal muscle",
INTERNATIONAL JOURNAL OF MOLECULAR
MEDICINE, vol. 37, no. 4, 12 February 2016
(2016-02-12), GR, pages 867 - 878, XP055692567,
ISSN: 1107-3756, DOI: 10.3892/ijmm.2016.2487**
• **EVELIEN WYNENDAELE ET AL: "Crosstalk
between the microbiome and cancer cells by
quorum sensing peptides", PEPTIDES, vol. 64, 1
February 2015 (2015-02-01), AMSTERDAM, NL,
pages 40 - 48, XP055373151, ISSN: 0196-9781,
DOI: 10.1016/j.peptides.2014.12.009**

Remarks:
The complete document including Reference Table(s) and the Sequence Listing(s) can be downloaded from the EPO website

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

Field of the invention

The present invention relates to diagnostic methods to assess the presence of quorum sensing molecules (QSM), preferably peptides (QSPs) that influence muscle wasting in humans. The present invention furthermore relates to the use of the knowledge obtained by the diagnostic method in order to influence muscle wasting diseases in animals and human, by for example providing bacteria that produce beneficial QSMs or non-harmful QSMs, providing antagonists for harmful QSMs

Background of the invention

Muscle wasting is an important clinical syndrome, that can be acute (*e.g.* critical illness myopathy), subacute (*e.g.* cancer cachexia) or chronic (*e.g.* sarcopenia) in onset. Although exercise, nutrition and hormonal therapies are currently recognized to have some beneficial effects on muscle wasting diseases, these interventions either require large efforts for relatively small health gains or have an unfavourable benefit/risk profile. Different biological systems have been demonstrated to play a role in muscle diseases but do differ in relative importance depending on the specific muscle disease. These cellular biological systems can be classified in five main groups: viability, differentiation, inflammation, mitochondrial changes and protein degradation. However, the upstream cascades and/or initiating triggers are currently not fully elucidated.

Quorum sensing molecules (QSM) are characteristic bacterial products, constitutively produced by living bacteria. The bacteria generally exhibit an increased production of QSMs in "stress" conditions.

Three main groups of quorum sensing molecules can be distinguished: the N-acyl homoserine lactones (AHL, mainly produced by Gram-negative bacteria), the quorum sensing peptides (QSP, mainly produced by Gram-positive bacteria) and the furanosyl borates (AI-2 [autoinducer-2], produced by Gram-positive as well as by Gram-negative bacteria). Moreover, a miscellaneous group of quorum sensing molecules with diverse structures (comprising amines, volatile compounds etc) is also described.

In addition to their intra-bacterial communication function, some quorum sensing molecules have already been demonstrated in in-vitro test systems to potentially cross the gut barrier and to be potential bacterial-host communication signals in for example cancer and central nervous system diseases. See for example: E. Wynendaele, F. Verbeke, M. D'Hondt, A. Hendrix, C. Van de Wiele, C. Burvenich, K. Peremans, O. De Wever, M. Bracke, B. De Spiegeleer, Crosstalk between the microbiome and cancer cells by quorum sensing peptides, Peptides 64 (2015) 40-48. B. De Spiegeleer, F. Verbeke, M. D'Hondt, A. Hendrix, C. Van De Wiele, C, Burvenich, K. Peremans, O. De Wever, M. Bracke, E. Wynendaele, The Quorum Sensing Peptides PhrG, CSP and EDF Promote Angiogenesis and Invasion of Breast Cancer Cells In Vitro, Plos One 10(3) (2015), and Y. Janssens, E. Wynendaele, F. Verbeke, N. Debunne, B. Gevaert, K. Audenaert, C. Van DeWiele, B. De Spiegeleer, Screening of quorum sensing peptides for biological effects in neuronal cells, Peptides 101 (2018) 150-156.

Bandyopadhaya et al. (A. Bandyopadhaya, C. Constantinou, N. Psychogios, R. Ueki, S. Yasuhara, J. Martyn, J. Wilhelmy, M. Mindrinos, L.G. Rahme, A.A. Tzika, Bacterial-excreted small volatile molecule 2-aminoacetophenone induces oxidative stress and apoptosis in murine skeletal muscle, International Journal of Molecular Medicine 37 (2016) 867-878; D1) provides one quorum sensing (QS) small volatile molecule, 2-aminoacetophenone (2-AA), excreted by *Pseudomonas aeruginosa* (PA), which is produced in infected human tissue, promotes bacterial phenotypes that favour chronic infection, while also compromising muscle function.

US 2015/0335688 A1 describes very general probiotics and small molecules in health diseases, however without any details of bacterial strains elected on specific QSM.

The present invention shows for the first time a role of quorum sensing molecules in muscle wasting diseases.

Summary of the invention

In one aspect, the invention is directed to an in vitro diagnostic method for analyzing the presence of QSMs in a sample of mammal to assess the presence of QSMs that influence muscle homeostasis, and in particular muscle wasting, and wherein the QSM is selected from the group consisting of: CVGIW (SEQ ID NO: 11; QSP022), ESRVSRIILDFLFQRKK (SEQ ID NO: 28; QSP055), KSSAYSLQMGATAIKQVKKLFKKWGW (SEQ ID NO: 40; QSP0125), SGSLSTFFRLFNRSFTQALGK (SEQ ID NO: 56; QSP176), and SIFTLVA (SEQ ID NO: 57; QSP184).

Screening can be done by analyzing feces and/or blood, other body-fluids, and/or other samples.

In a particular embodiment, disclosed herein is an in vitro method of diagnosing muscle wasting associated with cachexia, sarcopenia, physical frailty, critical illness myopathy, inflammatory myopathies, denervation or burns. The presence of the QSMs as provided herein, in particular Q022, Q055, Q125, Q176, Q184 and Q192, more in particular Q022, Q055, Q125, Q176 and Q184, in a sample of a subject is an indication of the presence or risk for muscle wasting associated with cachexia, sarcopenia, physical frailty, critical illness myopathy, inflammatory myopathies, denervation or burns.

The present invention furthermore relates to the use of the knowledge obtained by the diagnostic method in order to aid in the differential patient classification and in prognosis, as well as to influence the muscle wasting syndrome in animals and human, by for example providing bacteria that produce beneficial QSMs or non-harmful QSMs, providing antagonists for harmful QSMs.

The present invention in particular relates to life biotherapeutic products (LBP) or probiotica, having neutral or good QSM to replace in the gut the strains that have QSMs shown to negatively influence aspects of health.

Probiotica and LBP are known, and the present invention is also related to a method of screening bacterial species-strains suitable as probiotic or LBP strain, wherein the QSM is analysed, the found QSM is compared to the knowledge base for such QSM and the strain is qualified as positive, neutral or negative in relation to a certain disorder.

The invention furthermore relates to certain QSMs that are shown to influence muscle homeostasis, in particular muscle wasting. Some of the QSMs are shown to have a positive influence by increasing viability and/or differentiation, while others are shown to have a negative influence, for example by disturbing mitochondrial activity or increased inflammation. The latter class can be used to provide antagonist to such QSMs, and/or can be used to screen for bacterial strains that do not produce such QSMs. Moreover, hormesis effects can be expected to influence muscle wasting: also the concentration levels will influence the effects.

In addition to analyzing the presence of QSMs in a sample of mammal to assess the presence of QSMs that are known to influence muscle wasting, preferably, the diagnostic method described herein furthermore screens for additional disorders, such as for example colorectal cancer and/or breast cancer and/or psychiatric/neurological disorders: E. Wynendaele, F. Verbeke, M. D'Hondt, A. Hendrix, C. Van de Wiele, C. Burvenich, K. Peremans, O. De Wever, M. Bracke, B. De Spiegeleer, Crosstalk between the microbiome and cancer cells by quorum sensing peptides, Peptides 64 (2015) 40-48; B. De Spiegeleer, F. Verbeke, M. D'Hondt, A. Hendrix, C. Van De Wiele, C. Burvenich, K. Peremans, O. De Wever, M. Bracke, E. Wynendaele, The Quorum Sensing Peptides PhrG, CSP and EDF Promote Angiogenesis and Invasion of Breast Cancer Cells In Vitro, Plos One 10(3) (2015); Y. Janssens, E. Wynendaele, F. Verbeke, N. Debunne, B. Gevaert, K. Audenaert, C. Van De Wiele, B. De Spiegeleer, Screening of quorum sensing peptides for biological effects in neuronal cells, Peptides 101 (2018) 150-156.

Furthermore, the invention resides in providing antagonists of QSMs, for prophylactic or curative purposes. In one embodiment, the antagonists are peptides that share homology with the sequence of the respective QSMs.

In another aspect, the present disclosure provides a method of treating or preventing a muscle wasting in a subject by administering an effective amount of a the bacterial strains or antagonists as provided herein.

In one embodiment, muscles wasting is associated with primordial nongenetic, skeletal muscle wasting diseases.

In a further embodiment, the muscle wasting is associated with cachexia, sarcopenia, physical frailty, critical illness myopathy, inflammatory myopathies, denervation or burns.

Short description of the figures

**Figure 1.** Shows the viability and differentiation results of C2C12 myoblasts.

**Figure 2.** Effects of QSM on IL-6 production by C2C12 myotubes.

**Figure 3.** Effects of QSM on GDF15 production by C2C12 myotubes.

**Figure 4.** Protein degradation results of C2C12 myoblasts treated with QSM, using RT-qPCR.

**Figure 5.** Overall results of QSM influencing C2C12 cells on five biological systems.

**Figure 6A** and **6B** show the relative IL-6 secretion of Q055 (Fig 6A) and Q176 (Fig B) alanine-scan peptides.

**Figure 7** shows the relative MTT reduction of Q176 ala-scan peptides.

**Figure 8A, 8B and 8C** show respectively the IL-6 response of co-treatments of Q055 and derivatives (alascan-peptides), the MTT response of co-treatments of Q176 and derivatives (alascan-peptides), and the IL-6 response of co-treatments of Q176 and derivatives (alascan-peptides).

**Figure 9.** Dose-response viability curves of QSP022 and QSP 184 on human muscle cells.

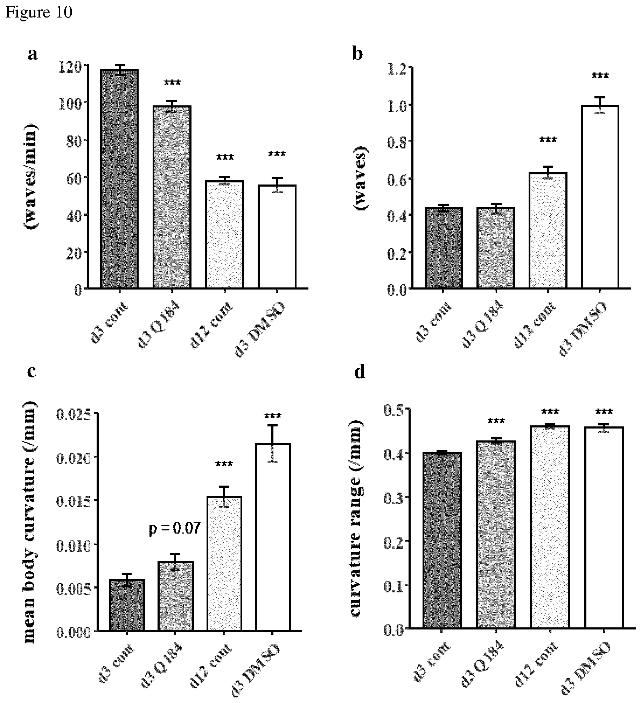

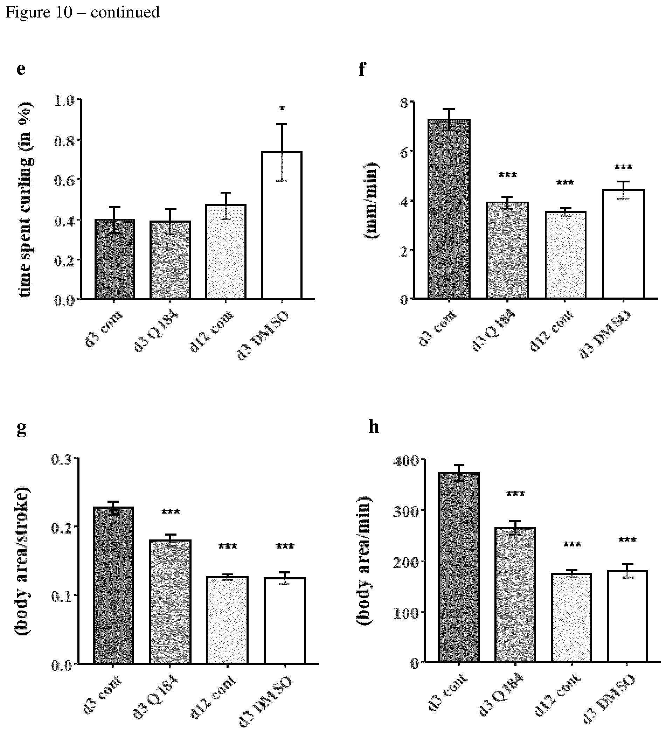

**Figure 10.** QSP184 induces an aging muscle phenotype in C. elegans. a-h, Q184-associated changes in swimming parameters in wild-type adults after 3 days with placebo control (d3 cont), 1 $\mu$M Q184 (d3 Q184) or DMSO (d3 DMSO), or after 12 days with placebo control (d12 cont). a, Wave initiation rate; b, Body wave number; c, Asymmetry; d, Stretch; e, Curling; f, Travel speed; g, Brush stroke; h, Activity index. Data presented are means $\pm$ s.e.m . * P < 0.05; ** P < 0.01 and *** P < 0.001; Welch's 2-sided t-test (n = 216 for 3d placebo control; n = 277 for 12d placebo control; n = 146 for 1 $\mu$M Q184; n = 155 for DMSO; from 5 independent trials).

**Figure 11.** QSP022 induces an aging muscle phenotype in C. elegans. a-h, QSP022-associated changes in swimming parameters in wild-type adults after 3 days with placebo control (Placebo), 1 $\mu$M QSP022 (QSP1), 10 $\mu$M QSP022 (QSP10) or DMSO 5% (DMSO). a, Wave initiation rate; b, Body wave number; c, Asymmetry; d, Stretch; e, Curling; f, Travel speed; g, Brush stroke; h, Activity index. Data presented are means $\pm$ s.e.m . * P < 0.05; ** P < 0.01 and *** P < 0.001; Welch's 2-sided t-test (from 5 independent trials).

**Figure 12.** Dose-response IL-6 curves of QSP055, QSP125 and QSP176 on human muscle cells.

**Figure 13.** Effect of QSP055, QSP125 and QSP176 on grip strength relative to baseline (T0) values. Grip strength was measured at baseline and after 2 weeks of daily IP injection of QSP (0.5 $\mu$mol/kg/d) or vehicle in 12-month-old mice. Boxplots represent median (midline), 25% and 75% percentiles (upper and lower perimeters) and maximum and minimum (tails, without outliers) (n = 9 for vehicle; n = 12 for QSP). Mean differences were analysed with Student's t-test.

Detailed description of the invention

Unless defined otherwise, all technical and scientific terms used herein have the same meaning as commonly understood to one of ordinary skill in the art to which this invention pertains. Otherwise, certain terms used herein have the meanings as set forth in the specification.

It must be noted that as used herein and in the appended claims, the singular forms "a," "an," and "the" include plural reference unless the context clearly dictates otherwise.

Unless otherwise stated, any numerical values, such as a concentration or a concentration range described herein, are to be understood as being modified in all instances by the term "about." Thus, a numerical value typically includes $\pm$ 10% of the recited value. For example, a concentration of 1 mg/mL includes 0.9 mg/mL to 1.1 mg/mL. Likewise, a concentration range of 1% to 10% includes 0.9% to 11 %. As used herein, the use of a numerical range expressly includes all possible subranges, all individual numerical values within that range, including integers within such ranges and fractions of the values unless the context clearly indicates otherwise.

As used herein, the terms "comprises," "comprising," "includes", "including," "has," "having," "contains" or "containing," or any other variation thereof, will be understood to imply the inclusion of a stated integer or group of integers but not the exclusion of any other integer or group of integers and are intended to be nonexclusive or open-ended. For example, a composition, or a process that comprises a list of elements is not necessarily limited to only those elements but can include other elements not expressly listed or inherent to such composition or method.

As used herein, the term "consists of," or variations such as "consist of or "consisting of," as used throughout the specification and claims, indicate the inclusion of any recited integer or group of integers, but that no additional integer or group of integers can be added to the specified method, structure, or composition.

As used herein, the term "consists essentially of," or variations such as "consist essentially of or "consisting essentially of," as used throughout the specification and claims, indicate the inclusion of any recited integer or group of integers, and the optional inclusion of any recited integer or group of integers that do not materially change the basic or novel properties of the specified method, structure or composition.

It should also be understood that the terms "about," "approximately," "generally," "substantially" and like terms, used herein when referring to a dimension or characteristic of a component of the preferred invention, indicate that the described dimension/characteristic is not a strict boundary or parameter and does not exclude minor variations therefrom that are functionally the same or similar, as would be understood by one having ordinary skill in the art. At a minimum, such references that include a numerical parameter would include variations that, using mathematical and industrial principles accepted in the art (e.g., rounding, measurement or other systematic errors, manufacturing tolerances, etc.), would not vary the least significant digit.

Muscle wasting (sometimes also referred to as muscle wasting syndrome) refers to the progressive loss of muscle mass and/or to the progressive weakening and degeneration of muscles, including skeletal or voluntary muscles, cardiac muscles controlling the heart (cardiomyopathies), and smooth muscles. Chronic muscle wasting is a condition (i.e., persisting over a long period of time) characterized by progressive loss of muscle mass, as well as muscle weakening and degeneration. The loss of muscle mass occurs when the rate of muscle protein degradation exceeds muscle protein synthesis. Muscle wasting is a debilitating and life-threatening disease state, which has been associated with the development of a number of acute and chronic, neurological, genetic, inflammatory, fibrotic or infectious pathologies, including, e.g, muscular dystrophies, amyotrophic lateral sclerosis, inflammatory myopathies, denervation muscle atrophies, neurological disease, cachexia, anorexia-cachexia syndrome, cancers, rheumatoid arthritis, osteoarthritis, diabetes, inflammatory bowel disease, liver cirrhosis, chronic obstructive pulmonary disease, pulmonary fibrosis, chronic renal disease, chronic heart failure, sarcopenia, physical frailty, androgen deprivation, corticosteroid myopathy, trauma (e.g. burns), critical illness myopathy (e.g. sepsis), acquired immune deficiency syndrome (AIDS) and cardiomyopathy.

In some embodiments, the present invention provides diagnostic and therapeutic methods as claimed related to muscle wasting associated with cachexia, sarcopenia, physical frailty, critical illness myopathy, inflammatory myopathies, denervation or burns.

Cachexia is a complex syndrome associated with an underlying illness causing ongoing muscle loss that is not entirely reversed with nutritional supplementation. A prominent clinical feature of cachexia is weight loss in adults (corrected for fluid retention) or growth failure in children. Cachexia can be caused by diverse medical conditions, but is most often associated with end-stage cancer, known as cancer cachexia, advanced chronic obstructive pulmonary disease, known as COPD cachexia, advanced chronic kidney disease, known as chronic kidney diseaseassociated cachexia, advanced chronic heart failure, known as cardiac cachexia, liver cirrhosis, known as hepatic cachexia, rheumatoid arthritis, known as rheumatoid cachexia, and AIDS, known as HIV-related cachexia.

Sarcopenia is a condition characterized by chronic loss of skeletal muscle mass and function, without overt underlying illness. Although it is primarily a disease of the elderly, its development may be associated with conditions that are not exclusively seen in older persons.

Physical frailty can be considered as pre-disability, with disability defined as needing assistance with basic Activities of Daily Living (ADL). Physical frailty is a muscle wasting syndrome with multiple causes and contributors, characterized by diminished strength, endurance and reduced physiologic functions that increases an individual's vulnerability to stressors and to develop increased dependency and/or death.

Critical illness myopathy is the rapidly evolving muscle wasting that occurs in critically ill patients. Often, but not exclusive, the patients have sepsis, systemic inflammatory response syndrome (SIRS) and/or multi-organ failure.

Inflammatory myopathies are a group of diseases, with no clear cause, that involve chronic muscle inflammation accompanied by muscle weakness. The three main types of inflammatory myopathy are polymyositis, dermatomyositis, and inclusion body myositis (IBM).

Screening

In one aspect, the invention is directed to an in vitro diagnostic method for analyzing the presence of QSMs in a sample of mammal to assess the presence of QSMs that influence muscle wasting, and wherein the QSM is selected from the group consisting of: CVGIW (SEQ ID NO: 11; QSP022), ESRVSRIILDFLFQRKK (SEQ ID NO: 28; QSP055), KSSAYSLQMGATAIKQVKKLFKKWGW (SEQ ID NO: 40; QSP0125), SGSLSTFFRLFNRSFTQALGK (SEQ ID NO: 56; QSP176), and SIFTLVA (SEQ ID NO: 57; QSP184).

The mammal can be human or an animal. A preferred species of a mammal is a human. Preferred animals are companion animals, such as cats or dogs. Other preferred animas are farm animals such as cattle, sheep or horse, preferably cattle or horse.

Screening can be done by analyzing feces and/or blood, other body-fluids, and/or other samples.

Some quorum sensing molecules have already been demonstrated to cross the gut barrier and to be potential bacterial-host communication signals in cancer and central nervous system diseases. Moreover, AI-2 has already been detected in human stool and saliva, while AHL have been detected in human sputum, urine and plasma. QSP, a relatively unexplored group of QSM in the context of microbiome-host interactions, have very recently unambiguously been demonstrated to be present in mice plasma.

Suitable analytical techniques are for example described in: F. Verbeke et al., Peptides as quorum sensing molecules: measurement techniques and obtained levels in vitro and in vivo, Front. Neurosci. 11 (2017)183; F. Verbeke et al., Journal of Pharmaceutical and Biomedical Analysis 160 (2018) 55-63, and N. Debunne et al. Chromatographia (2018) 81:25-40.

The method used for analysing the quorum sensing peptides generally consists of a gradient system (UPLC or HPLC), coupled to mass spectrometry peptides. Suitable examples include one or more of the following: A suitable column is an Acquity UPLC® BEH300 C18 1.7$\mu$m; 2.1 × 100 mm (U2), using as solvents: as mobile phase A: 95/5 $H_2O$/ACN + 0.1% formic acid and Mobile phase B: 5/95 $H_2O$/ACN + 0.1% formic acid with a column temperature of 60°C. The MS is set at scan mode between 300-1250 m/z

Another suitable column is Phenomenex LC Jupiter® H17-209184 C18 5$\mu$m (250 × 4.6 mm), using comparable solvents

Another suitable column is a C18 column, Acquity UHPLC BEH300 C18 (2.1 × 100 mm; 1.7 $\mu$m) combined with a guard column (column e.g. n° U-2); mobile phases: A, water/ACN/DMSO 93/2/5 (V/V/V) + 0.1% FA (m/V); B: water/ACN/DMSO 2/93/5 (V/V/V) + 0.1% FA (m/V).

Another suitable column is an HILIC amide column: Acquity UHPLC BEH Amide (2.1 × 100 mm; 1.7 $\mu$m) combined with a guard column (column e.g. n° U-18) using comparable mobile phase compositions.

The UHPLC system was connected to the Xevo™ TQ-S triple quadrupole mass spectrometer with electrospray ionisation (operated in the positive ionisation mode).

A preferred diluent is obtained as follows: approximately 0.5 g BSA is dissolved in 50 mL $H_2O$ + 0.1% FA. 25 mL of this solution is transferred into a 100 mL volumetric flask and diluted ad 100 mL with ACN + 0.1% FA. This solution is transferred to 20 2.0 mL protein lobind tubes. The solution is heated to 95°C for 5 minutes and cooled down for 30 minutes on ice. Next, the tubes are centrifuged for 15 minutes at 20000g at 5°C. 33.3 mL supernatant was diluted ad 50.0 mL with $H_2O$ + 0.1% FA. The obtained DruQuaR diluent is stored for a maximum of 14 days at 6°C.

This diluent generally was used to coat the vials in which the QSM, in particular the QSP, was processed.

In a further aspect, described herein is a method of preventing, risk evaluation, diagnosis, treating and/or reducing muscle wasting in a subject, said method comprising the steps of:

- detecting the presence and level of one or more selected QSMs in a sample derived from said subject;
- based on the outcome, which can also serve as a diagnostic biomarker, adjusting the diet and/or lifestyle of said subject to prevent and/or reduce the uptake and/or presence of a bacterial strain producing said QSM, as provided in the claims.

Methods of treatment

The present invention furthermore relates to the use of the knowledge obtained by the diagnostic method in order to influence muscle wasting diseases/syndrome in animals and human, by providing bacteria from the species Lactobacillus plantarum, Enterococcus faecalis, Streptococcus mitis or Streptococcus mutans that produce beneficial QSMs or non-harmful QSMs, or by providing the peptide antagonists for the harmful QSMs disclosed herein. Next to the possibility of influencing muscle wasting, other disorders that are, or will appear to be influenced negatively by QSMs can be treated as a result of this diagnostic method.

In one embodiment, the invention provides (beneficial) bacterial strains from the species Lactobacillus plantarum, Enterococcus faecalis, Streptococcus mitis or Streptococcus mutans for use in preventing, slowing down, inhibiting or treating muscle wasting, wherein the bacterial strain is not producing one or more of the QSMs of the invention identified to have a negative impact on muscle homeostasis and/or to induce muscle wasting, i.e. Q022, Q055, Q125, Q176, and Q184. More specific, such a bacterial strain does not produce one or more of said QSMs. In particular, the bacterial strain is lacking the QSM gene or is genetically modified e.g. by deletion or mutation of the respective QSM gene. Typically, the genetic modification results in a non-functional QSM gene, e.g. by mutation or deletion in the QSM gene (such as e.g. the nucleotide sequences translated into peptidic QSMs), in particular a deletion of the full QSM gene. As a further option, the bacterial strain is a wild-type strain that does not produce a functional QSMs of the invention identified to have a negative impact on muscle homeostasis and/or to induce muscle wasting, i.e. Q022, Q055, Q125, Q176, and Q184. More specific, such a bacterial strain does not produce one or more of the QSMs selected from the group consisting of: CVGIW (SEQ ID NO: 11; QSP022), ESRVSRIILDFLFQRKK (SEQ ID NO: 28; QSP055), KSSAYSLQMGATAIKQVKKLFKKWGW (SEQ ID NO: 40; QSP0125), SGSLSTFFRLFNRSFTQALGK (SEQ ID NO: 56; QSP176), and SIFTLVA (SEQ ID NO: 57; QSP184).

In general and as known to the skilled person, different nucleotide sequences are translated into peptidic QSM. Exemplary nucleotide sequences translating into Q022, Q054, Q055, Q125, Q176, Q184 or Q192 are shown here below.

| QSM | nucleotide sequence (5' -> 3') | SEQ ID NO |
|---|---|---|
| Q022 | TGTGTAGGAATTTGG | 81 |
| Q054 | GAAAGTAGACTGCCAAAAATCCGATTTGATTTTATT TTCCCACGAAAAAAG | 82 |
| Q055 | GAAAGTAGGGTTTCAAGAATCATCCTTGATTTTCTT TTCCAACGAAAAAAG | 83 |
| Q125 | AAGAGTAGTGCGTATTCTTTGCAGATGGGGGCAAC TGCAATTAAACAGGTAAAGAAACTGTTTAAAAAAT GGGGATGG | 84 |
| Q176 | AGCGGAAGCCTATCAACATTTTTCCGGCTGTTTAAC AGAAGTTTTACACAAGCTTTGGGAAAATAA | 85 |
| Q184 | AGTATTTTTACTTTAGTAGCA | 86 |
| Q192 | TCAAGGAATGCAACATGA | 87 |

Absence of a specific gene and/or its QSM product can be determined by any technique known to the skilled person, such as for example qPCR and/or UHPLC-MS/MS and/or any other established technique. In a further embodiment, the bacterial strain is from the species *Lactobacillus plantarum, Enterococcus faecalis, Streptococcus mitis* or *Streptococcus mutans.* Suitable strains as proof-of-concept are identified in Table 4 and 5. Hence, the invention provides these bacterial strains for use in preventing, treating or reducing muscle wasting associated with muscular dystrophies, amyotrophic lateral sclerosis, inflammatory myopathies, denervation muscle atrophies, neurological disease, cachexia, anorexia-cachexia syndrome, cancers, rheumatoid arthritis, osteoarthritis, diabetes, inflammatory bowel disease, liver cirrhosis, chronic obstructive pulmonary disease, pulmonary fibrosis, chronic renal disease, chronic heart failure, sarcopenia, physical frailty, androgen deprivation, corticosteroid myopathy, trauma (e.g. burns), critical illness myopathy (e.g. sepsis), acquired immune deficiency syndrome (AIDS) or cardiomyopathy.The present invention in particular relates to live biotherapeutic products or medicine (LBP; product containing live microorganisms that is applicable to the prevention, treatment, or cure of a disease or condition in human beings) and probiotica (live microorganisms that when administered in adequate amounts confer a health benefit on the host e.g. healthy for muscles), having neutral or good QSM, as claimed, to replace in the gut the strains that have QSMs shown to negatively influence aspects of health, like in particular muscle homeostasis, more in particular wasting. Hence the strain that does not produce the QSM of the invention is administered to the subject and reduces the production of the resp. QSM by bacterial strains in the gastro-intestinal tract e.g. by altering the bacterial flora and/or suppressing the levels of the QSM producing strains. In other words, in one aspect of the invention, administration of the bacterial strains reduces the production of the QSM by other bacterial strains in the gastro-intestinal tract. The bacterial strains can be administered to the subject orally, in all possible forms such as formulated in a powder, tablet, capsule, or any other way known to the skilled person. In one embodiment, the bacterial strain is administered as part of a composition, for example via the daily food or via a specific pharmaceutical composition.

Probiotica and LBP are known, and the present invention is also related to a method of screening bacterial strains suitable as probiotic or LBP strain, wherein the QSM produced by said bacterial strain, said QSM selected from the group consisting of: CVGIW (SEQ ID NO: 11; QSP022), ESRVSRIILDFLFQRKK (SEQ ID NO: 28; QSP055), KSSAYSLQMGATAIKQVKKLFKKWGW (SEQ ID NO: 40; QSP0125), SGSLSTFFRLFNRSFTQALGK (SEQ ID NO: 56; QSP176), and SIFTLVA (SEQ ID NO: 57; QSP184), is analysed and determined, the found QSM is compared to the knowledge base for QSM's and the strain is qualified as positive, neutral or negative in relation to a certain disorder. Herewith, a tool is provided to select probiotic bacterial strains that release QSMs that are at least non-harmful, or preferably are providing positive effects on disorders in mammals, preferably human. In one embodiment, the bacterial strain is from the genus *Lactobacillus, Enterococcus* or *Staphylococcus,* and in particular from the species *Lactobacillus plantarum, Enterococcus faecalis, Streptococcus mitis* or *Streptococcus mutans.*

Furthermore, the invention resides in providing peptide antagonists of QSM's, having the following characteristics: the peptide antagonist of QSP055 (SEQ ID NO: 28) is a peptide having the amino acid sequence SRVSAIILDFLFQRKK (SEQ ID NO: 77; QSP055-A6) or the amino acid sequence ESRVSRIIADFLFQRKK (SEQ ID NO: 78; QSP055-A9), and the peptide antagonist of QSP176 (SEQ ID NO: 56) is a peptide having the amino acid sequence GSLSTFFALFNRSFTQALGK (SEQ ID NO: 79; QSP176-A9) or the amino acid sequence GSLSTFFRLFNASFTQALGK (SEQ ID NO: 80;QSP176-A13). In one embodiment, the invention provides these peptide antagonists for use as a medicine. Such antagonist can be found by commonly applied screening techniques. Suitable agonists or antagonists include for example analogues of QSMs that comprise one or more amino acids or amino acid derivatives. In one embodiment, the antagonists are peptide antagonists having an amino acid sequence homologous to the respective QSP selected from the group consisting of: CVGIW (SEQ ID NO: 11; QSP022), ESRVSRIILDFLFQRKK (SEQ ID NO: 28; QSP055), and KSSAYSLQMGATAIKQVKKLFKKWGW (SEQ ID NO: 40; QSP0125), SGSLSTFFRLFNRSFTQALGK (SEQ ID NO: 56; QSP176), and SIFTLVA (SEQ ID NO: 57; QSP184), i.e. having one or two deleted or substituted amino acids, and are for use in preventing, reducing or treating muscle wasting associated with muscular dystrophies, amyotrophic lateral sclerosis, inflammatory myopathies, denervation muscle atrophies, neurological disease, cachexia, anorexia-cachexia syndrome, cancers, rheumatoid arthritis, osteoarthritis, diabetes, inflammatory bowel disease, liver cirrhosis, chronic obstructive pulmonary disease, pulmonary fibrosis, chronic renal disease, chronic heart failure, sarcopenia, physical frailty, androgen deprivation, corticosteroid myopathy, trauma (e.g. burns), critical illness myopathy (e.g. sepsis), acquired immune deficiency syndrome (AIDS) or cardiomyopathy.-Specific examples of suitable peptide antagonists useful in the methods of the present invention are SRVSAIII,DFLFQRKK (SEQ ID NO: 77; QSP055-A6), ESRVSRIIADFLFQRKK (SEQ ID NO: 78; QSP055-A9), GSLSTFFALFNRSFTQALGK (SEQ ID NO: 79; QSP176-A9), and GSLSTFFRLFNASFTQALGK (SEQ ID NO: 80; QSP176-A13).

With the terms "preventing", "inhibiting" or "treating" is meant any treatment of a disease and/or condition in a subject, and includes: (i) preventing a disease and/or condition from occurring in a subject which may be predisposed to the disease and/or condition but has not yet been diagnosed as having it; (ii) inhibiting the disease and/or condition, i.e., slowing down or arresting its development; (iii) relieving the disease and/or condition, i.e., causing regression of the disease and/or condition.

Generally, for pharmaceutical use, the bacterial strains or QSM agonists/antagonists of the invention may be formulated as a pharmaceutical preparation or pharmaceutical composition comprising at least one bacterial strain or QSM agonist/antagonist of the invention and at least one pharmaceutically acceptable carrier, diluent or excipient and/or adjuvant, and optionally one or more further pharmaceutically active compounds. Such a formulation may be in a form suitable for oral administration, or for administration by suppository, or for parenteral administration (such as by intravenous, intramuscular or subcutaneous injection or intravenous infusion), for intranasal, transdermal, transmucosal, rectal or pulmonary administration. Examples of such formulations include tablets, pills, powders, lozenges, sachets, cachets, elixirs, suspensions, emulsions, solutions, syrups, aerosols, ointments, creams, lotions, soft and hard gelatin capsules, suppositories, eye drops, sterile injectable solutions and sterile packaged powders (which are usually reconstituted prior to use) for administration as a bolus and/or for continuous administration. Carriers, excipients, and diluents that are suitable for such formulations are e.g. lactose, dextrose, sucrose, sorbitol, mannitol, starches, gum acacia, calcium phosphate, alginates, tragacanth, gelatin, calcium silicate, polyvinylpyrrolidone, polyethylene glycol, cellulose and its variants (eg microcrystalline) and derivatives (eg hypromellose), (sterile) water, methylcellulose, methyl- and propylhydroxybenzoates, talc, magnesium stearate, edible oils, vegetable oils and mineral oils or suitable mixtures thereof.

This invention further provides a pharmaceutical composition comprising a bacterial strain or QSM agonist/antagonist according to this invention and a pharmaceutically acceptable carrier, diluent and/or excipient, and the uses thereof as provided herein.

QSMs influencing muscle wasting

Disclosed herein are certain QSMs that are shown to influence muscle homeostasis, in particular muscle wasting. Some of the QSMs are shown to have a positive influence, for example by increasing viability and/or differentiation, while others are shown to have a negative influence, for example by decreasing viability and/or differentiation, disturbing mitochondrial activity or increased inflammation.

The latter class can be used to provide (peptide) antagonist to such QSMs, and/or can be used to screen for bacterial strains that do not produce such QSMs.

Moreover, hormesis effects can be expected: also the concentration levels will influence the effects. For example it is known that local low dose inflammation or IL-6 production has beneficial effects on muscle, while systemic high dose inflammation or IL-6 production is detrimental. Reviews referring to the hormesis effects of IL-6 are for example: C. Brandt et al., Journal of Biomedicine and Biotechnology (2010) ID 520258, and S. Welc et al., Exp. Physiol. 98(2) (2013) 359-371.

The QSMs influencing muscle wasting and of particular interest in the uses and methods described herein include the following: AI-2, 3OH-C6-HSL, C6-HSL, Q007, Q011, Q015, Q017, Q018, Q019, Q022, Q030, Q031, Q040, Q052, Q054, Q055, Q093, Q097, Q099, Q101, Q125, Q132, Q134, Q138, Q140, Q146, Q164, Q176, Q184 and Q192.

Preferred QSMs influencing muscle wasting include the following: AI-2, Q022, Q054, Q055, Q125, Q176, Q184 and Q192. Said QSMs have been shown for the first time in the present invention to have an impact on muscle homeostasis such as muscle viability, differentiation, inflammation, mitochondrial changes and/or protein degradation.

Preferred QSPs influencing muscle wasting include the following: Q022, Q054, Q055, Q125, Q176, Q184 and Q192, and more specific Q022, Q055, Q125, Q176, and Q184 (negative influence) or Q054 and Q192 (positive influence).

Experiments and discussion

**Screening experiments**

Using C2C12 myoblasts and 75 quorum sensing molecules, QSMs are identified with effects on muscle homeostasis, such as viability, differentiation, inflammation, mitochondrial changes or protein degradation.

Study design

75 QSM (68 QSP, 5 AHL, AI-2 and 2-AA) were screened for their activity on C2C12 cells *in vitro* using 5 assays (viability, differentiation, inflammation, mitochondrial changes, protein degradation). Each QSM was independently studied twice in every assay (n=2 biological replicates). For the protein degradation, a tiered approach was used for feasibility reasons, *i.e.* QSM were first screened in pairs (n=2 for each pair) and the possible hits were then screened individually (n=2 for each QSM).

Quorum sensing molecules

All molecules were purchased demanding a purity of minimally 95%. The solvents used were water or water + dimethylsulfoxide. The QSM's other than QSPs used for screening are described in Table 1.

**Table 1**

| Abbreviation | Molecular Formula | Mw (Da) |
|---|---|---|
| C8-L-HSL | $C_{12}H_{21}NO_3$ | 227.30 |
| C12-L-HSL | $C_{16}H_{29}NO_3$ | 283.41 |
| C6-HSL | $C_{10}H_{17}NO_3$ | 199.20 |
| 3-OH-C6-HSL | $C_{10}H_{17}NO_4$ | 215.25 |
| 3-oxo-C12-HSL | $C_{16}H_{27}NO_4$ | 297.40 |
| AI-2 | $C_5H_{10}BO_7$ | 192.94 |
| 2-AA | $C_8H_9NO$ | 135.17 |

The QSPs used for screening are described in Table 2

**Table 2**

| Quorumpeps ID | Sequence | Mw(Da) | SEQ ID NO |
|---|---|---|---|
| 7 | YSPWTNF | 913.11 | 1 |
| 10 | ADLPFEF | 837.93 | 2 |
| 11 | AGTKPQGKPASNLVECVFSLFKKCN | 2667.14 | 3 |
| 13 | AIFILAS | 733.91 | 4 |
| 14 | AITLIFI | 790.01 | 5 |
| 15 | AKDEH | 598.61 | 6 |
| 16 | AKTVQ | 545.64 | 7 |
| 17 | ALILTLVS | 829.05 | 8 |
| 18 | ARNQT | 588.62 | 9 |
| 19 | NNWNN | 660.64 | 10 |
| 22 | CVGIW, thiolacton linkage between C1 and W5 | 558.78 | 11 |
| 28 | DIRHRINNSIWRDIFLKRK | 2480.91 | 12 |
| 30 | DLRGVPNPWGWIFGR | 1770.03 | 13 |
| 31 | DLRNIFLKIKFKKK | 1791.26 | 14 |
| 34 | DRVGA | 516.55 | 15 |
| 40 | DSVCASYF, thiolacton linkage between C4 and F8 | 873.06 | 16 |
| 42 | DWRFLNSIRDLIFPKRK | 2204.61 | 17 |
| 44 | EKMIG | 576.71 | 18 |
| 45 | EMRISRIILDFLFLRKK | 2178.71 | 19 |
| 46 | EMRKSNNNFFHFLRRI | 2109.44 | 20 |
| 47 | EMRLPKILRDFIFPRKK | 2187.72 | 21 |
| 49 | EQLSFTSIGILQLL TIGTRSCWFFYCR Y | 3346.92 | 22 |
| 50 | ERGMT | 592.67 | 23 |
| 51 | ERNNT | 632.63 | 24 |
| 52 | ERPVG | 556.62 | 25 |
| 53 | ESRLPKILLDFLFLRKK | 2116.62 | 26 |
| 54 | ESRLPKIRFDFIFPRKK | 2177.62 | 27 |
| 55 | ESRVSRIILDFLFQRKK | 2135.54 | 28 |
| 56 | VNYGNGVSCSKTKCSVNWGQAFQER YTAGINSFVSGVASGAGSIGRRP | 4969.46 | 29 |
| 58 | DSRIRMGFDFSKLFGK | 1904.22 | 30 |
| 62 | ESRISDILLDFLFQRKK | 2108.47 | 31 |
| 92 | FHWWQTSPAHFS | 1530.66 | 32 |

(continued)

| Quorumpeps ID | Sequence | Mw(Da) | SEQ ID NO |
|---|---|---|---|
| 93 | FLVMFLSG | 913.14 | 33 |
| 97 | QNSPNIFGQWM, lacton linkage between S3 and M11 | 1303.63 | 34 |
| 99 | GKAEF | 550.61 | 35 |
| 101 | GLWEDILYSLNIIKHNNTKGLHHPIQL | 3167.67 | 36 |
| 102 | GLWEDLLYNINRYAHYIT | 2254.53 | 37 |
| 121 | ILSGAPCIPW | 1056.29 | 38 |
| 123 | IRFVT | 634.78 | 39 |
| 125 | KSSAYSLQMGATAIKQVKKLFKKWGW | 2985.59 | 40 |
| 131 | KYYPCFGYF, thiolacton linkage between C5 and F9 | 1169.5 | 41 |
| 132 | LFSLVLAG | 819.01 | 42 |
| 133 | LFVVTLVG | 847.06 | 43 |
| 134 | LPFEF | 651.76 | 44 |
| 135 | LPFEH | 641.72 | 45 |
| 137 | LVTLVFV | 790.01 | 46 |
| 138 | MAGNSSNFIHKIKQIFTHR | 2229.59 | 47 |
| 140 | MKAEH | 614.72 | 48 |
| 143 | MPFEF | 669.79 | 49 |
| 146 | NEVPFEF | 880.95 | 50 |
| 148 | YSTCDFIM, thiolacton linkage between C4 and M8 | 961.24 | 51 |
| 160 | QKGMY | 625.74 | 52 |
| 162 | QRGMI | 603.74 | 53 |
| 164 | SDLPFEH | 843.89 | 54 |
| 165 | SDMPFEF | 871.96 | 55 |
| 176 | SGSLSTFFRLFNRSFTQALGK | 2364.69 | 56 |
| 184 | SIFTLVA | 749.9 | 57 |
| 186 | SKDYN | 625.64 | 58 |
| 191 | SRKAT | 561.64 | 59 |
| 192 | SRNAT | 547.57 | 60 |
| 193 | SRNVT | 575.62 | 61 |
| 206 | SYPGWSW | 881.94 | 62 |
| 208 | TNRNYGKPNKDIGTCIWSGFRHC | 2668.01 | 63 |
| 210 | VAVLVLGA | 740.94 | 64 |
| 212 | VPFEF | 637.73 | 65 |
| 214 | WPFAHWPWQYPR | 1670.89 | 66 |
| 218 | YNPCSNYL, thiolacton linkage between C4 and L8 | 955.18 | 67 |
| 298 | ILIVGG | 683.89 | 68 |

The peptides (68 in total) were labelled by their Quorumpeps ID number. Both non-peptide and peptide QSM were used in a physiologically and exposure relevant final concentration of 1 μM in all screening experiments, prepared from a 1 mg/mL stock solution.

C2C12 cell culture

The mouse myoblast C2C12 cell line (ATCC, CRL-1772) was cultured at 37°C in a 5% $CO_2$ humidified incubator in Dulbecco's modified Eagle medium (DMEM; Thermo Fisher Scientific). For C2C12 cell proliferation, the medium was supplemented with 10% fetal bovine serum (FBS; Thermo Fisher Scientific), 100 units/ml penicillin, and 100 μg/ml streptomycin (1% Pen/Strep; Thermo Fisher Scientific). The proliferation medium was renewed each 2-3 days and confluency was kept below 80%. For differentiation of the C2C 12 myoblasts into myotubes, proliferation medium was replaced by differentiation medium when cells reached 80% confluency. Differentiation medium consisted of DMEM supplemented with 2% horse serum (HS; Thermo Fisher Scientific) and 1% Pen/Strep. All experiments were done with C2C12 passage numbers below 30.

Cell viability

Viability of QSM was assessed using the 3-(4,5-dimethylthiazol-2-yl)-2,5-diphenyltetrazolium bromide (MTT) assay. Briefly, C2C12 myoblasts were seeded in a 96-well plate ($3 \times 10^4$ cells/well). 24 hours post-seeding, the wells were treated with QSM (1 $\mu$M), placebo or positive control (DMSO 2%) and incubated for 24 hours at 37°C. Next, 20 $\mu$L of MTT reagent (5 mg/mL; Sigma-Aldrich) was added to each well and the plates were returned to the incubator for 2.5 hours. After incubation, supernatant was removed from the wells and 150 $\mu$L of DMSO was added to release and dissolve the formazan crystals. Absorbance was measured at 570 nm using a microplate reader (Thermo Fisher).

Differentiation

C2C12 myoblasts ($1 \times 10^4$ cells/well) were seeded in 48-well plates and incubated until they reached 80% confluency, after which the proliferation medium was removed and replaced by differentiation medium ($d_0$). The differentiation medium was renewed at $d_3$. QSM (1 $\mu$M), placebo or positive controls (vitamin $D_3$) were added to the cells together with the differentiation medium at $d_0$ and $d_3$. After 6 days of incubation ($d_6$), myotubes were stained with the combined nuclear and cytoplasmatic LADD Multiple Stain (as described in R. McColl, M. Nkosi, C. Snyman, C. Niesler, Analysis and quantification of in vitro myoblast fusion using the LADD Multiple Stain, Biotechniques 61(6) (2016) 323-326). LADD Multiple Stain is a solution containing toluidine blue (VWR) and basic fuchsin (Fluka) in 30% (v/v) ethanol. The stained myotubes were assessed under a phase-contrast microscope equipped with a camera (Olympus CKX53) at a total magnification of 100x. Five different images were taken per well. The amount of myotube formation was automatically quantified using ImageJ software.

Inflammation

C2C12 myoblasts ($2.5 \times 10^4$ cells/well) were seeded in 96-well plates and incubated until they reached 80% confluency, after which the proliferation medium was removed and replaced by differentiation medium. After 6 days of incubation with daily renewed differentiation medium, the cells were treated with 1 $\mu$M of QSM, placebo or positive control (LPS) for 24 hours. IL-6 levels were determined in the supernatants using a sandwich ELISA (eBioscience), using IL-6 capture antibody, blocking solution, samples and standards, IL-6 detection antibody, avidin-horseradish peroxidase and substrate solution which were added sequentially, with washing steps in between. After stopping the reaction with $H_2SO_4$ solution, the absorbance was measured at 450 nm and 570 nm (correction wavelength) using a microplate reader (ThermoLab Systems). Concentrations were determined using the standard curves generated with known concentrations of IL-6.

Mitochondrial changes

GDF15 levels were determined in the supernatants of C2C12 cells after QSM, placebo or positive control (oligomyin) treatment, using a sandwich ELISA (R&D Systems). A similar protocol as described for the IL-6 determination was used.

Protein degradation

C2C12 cells were seeded in 6-well ($4 \times 10^5$ cells/well) or 12-well plates ($1.5 \times 10^5$ cells/well). 24 hours post-seeding, the wells were treated with QSM (1 $\mu$M), placebo or positive control (dexamethasone and LPS) solutions and incubated for 24 hours at 37°C. Thereafter, cells were scraped off from the well surface and resuspended in RLT lysis buffer (Qiagen). RNA was extracted from these lysed cells using RNeasy micro columns (Qiagen). Subsequently, RNA was reverse-transcribed using a mix of oligo-dT and random primers together with Quantiscript reverse transcriptase (Qiagen). DNAse steps were included in the protocol to remove possible DNA contamination. Real-time PCR was performed starting with 7.5 ng cDNA and both sense and antisense oligonucleotides in a final volume of 8 $\mu$l in a 384-well plate using the SensiMix SYBR green PCR master mix (Bioline). The following genes were analysed: atrogin, MuRF1, caspase 3 and atg7.

Table 3 shows the primer sequences (Invitrogen) for the different genes. The second derivative method was used to determine the threshold or Cq values, with analysis of *Ppia* (Peptidylprolyl isomerase A), *Rer* (Rhodopsin enhancer region) and *Hprt* (Hypoxanthine-guanine phosphoribosyltransferase) mRNA to normalize gene expression (delta Cq method). To ensure adequate precision and accuracy of the results, quality control steps were included at the RNA extraction level (purity, integrity) and the PCR level (specificity, efficiency).

**Table 3. Primer sequences of the protein degradation genes.**

|  | Sequence (5'-> 3') |
|---|---|
| Atrogin | Fw: TGAATAGCATCCAGATCAGC (SEQ ID NO 69)<br>Rev: CCTCTCTGAGAAGTGGTACT (SEQ ID NO 70) |
| MuRF | Fw: AAGACTGAGCTGAGTAACTG (SEQ ID NO 71)<br>Rev: GTAGAGGGTGTCAAACTTCT (SEQ ID NO 72) |
| Caspase 3 | Fw: TGTATGCTTACTCTACAGCAC (SEQ ID NO 73)<br>Rev: AAGGACTCGAATTCCGTTG (SEQ ID NO 74) |
| Atg7 | Fw: CATCTTCCTGCTAATGGACA (SEQ ID NO 75)<br>Rev: AAAGGTATCAAACCCCAAGG (SEQ ID NO 76) |

Statistical analysis

The following steps were consequently followed when analysing and interpreting the data: 1) data normalisation, 2) QC of the data, 3) hit identification.

Data normalisation

To make the two biological replicates of each assay comparable, the raw data were normalized with the median of each experiment as described in X.H.D. Zhang, Illustration of SSMD, z Score, SSMD*, z* Score, and t Statistic for Hit Selection in RNAi High-Throughput Screens, Journal of Biomolecular Screening 16(7) (2011) 775-785 and T. Valikangas, T. Suomi, L.L. Elo, A systematic evaluation of normalization methods in quantitative label-free proteomics, Briefings in Bioinformatics 19(1) (2018) 1-11:

$$x_i = \frac{r_i}{median(r_n)}$$

with $x_i$ being the $i^{th}$ normalized data point, $r_i$ the $i^{th}$ raw data point and $r_n$ all the raw data points for a given experiment (positive controls excluded). This operation is allowed, presuming that most of the tested compounds will be inactive for a given experiment and that the two biological replicates of each assay differ only by a constant. The median instead of mean was used because the median is less influenced by outliers (*i.e.* active compounds).

*QC of the data*

Before analysing the data in depth and identifying the QSM hits, the quality of the assay and of the corresponding data was first evaluated using positive and negative (*i.e.* placebo) controls, which were present in each assay. This was done by calculating the strictly standardized mean difference (SSMD) according to the following formula [17]:

$$SSMD = \frac{\overline{x_+} - \overline{x_-}}{\sqrt{s_+^2 + s_-^2}}$$

with s+ and x+ representing the standard deviation and the mean of the positive control values respectively, and s_ and x_ representing the standard deviation and the mean of the negative controls respectively. An assay was considered to be of sufficient quality if the absolute value of the SSMD was higher than 0. 5.

*Hit identification*

The obtained normalized data from the different assays were evaluated for a possible hit using four approaches as described in X.H.D. Zhang, Illustration of SSMD, z Score, SSMD*, z* Score, and t Statistic for Hit Selection in RNAi High-Throughput Screens, Journal of Biomolecular Screening 16(7) (2011) 775-785 and X.H.D. Zhang, X.T.C. Yang, N.J. Chung, A. Gates, E. Stec, P. Kunapuli, D.J. Holder, M. Ferrer, A.S. Espeseth, Robust statistical methods for hit selection in RNA interference high-throughput screening experiments, Pharmacogenomics 7(3) (2006) 299-309.

Every QSM that was scored as a hit using one of the four approaches was retained as a hit. QSM that were scored as a hit by at least three approaches, were defined as strong hits. The following approaches were used:

(i) Median absolute deviation (MAD):
While the mean and standard deviation (SD) are sensitive to outliers and violation of normality, the median and MAD are more robust measurements. The MAD was calculated according to the following formula:

$$MAD = 1.4826 \, M_i \left( \left| x_i - M_j \left( x_j \right) \right| \right)$$

with $M_j(x_j)$ the median of the normalized data points $x_j$, $x_i$ the $i^{th}$ normalized data point and $M_i(|x_i - M_j(x_j)|)$ the median of the absolute deviations between $x_i$ and $M_j(x_j)$. Similar to the classical hit-selection using mean $\pm$ k SD, cut-off values in the robust MAD method are generated using median $\pm$ k MAD. K was set to be 2, *i.e.* values outside the median $\pm$ 2MAD interval were considered to be outliers and thus hits.

(ii) Quartile-based method:
With this method, hits were selected as compounds being more extreme then cut-off values based on the quartiles and interquartiles of the data. The following formulae were used to define the cut-off values:

$$LCO = Q_1 - 1.9652 \left( Q_2 - Q_1 \right)$$

$$UCO = Q_3 - 1.9652 \left( Q_3 - Q_2 \right)$$

with LCO representing the lower cut-off, UCO the upper cut-off and Q1, Q2 and Q3 the lower, median and upper quartile, respectively. The major advantage using this method is its robustness against non-symmetrical data.

(iii) SSMD
Because most of the test samples had two biological replicate data points, this variability information was included in our hit-selection by using the SSMD approach. For this purpose, the average and standard deviation of the normalized data for each sample were used. To have a better estimate of the real standard deviation, the median standard deviation of all samples was also used. When n=1 in exceptional cases, only this median standard deviation was used. The following formula was used to calculate SSMD:

$$SSMD = \frac{\overline{x_i}}{\sqrt{w_i s_i^2 + w_0 s_0^2}}$$

with $\overline{x_i}$ and $s_i$ being the sample mean and sample standard deviation respectively, while $s_o$ represents the median standard deviation of all samples. The weights $w_i$ and $w_o$ were set to be 0.5. If the SSMD was above 3, the sample was selected as a hit.

(iv) Robust SSMD
Similar calculations and hit selection as the SSMD, but instead of using mean and standard deviation, the more robust median and MAD are used.

The circular bar plots and the overall hit-selection plot in this manuscript were made in RStudio 3.5.3. In the circular bar plots, the overall bar heights represent the mean of normalised data (n=2), *i.e.* median effect = 100%. The light grey bar represents the first result divided by two; the dark grey bar represents the second result divided by two. If the two bars are of equal length, there is no variability between the two biological replicates. When n=1 in exceptional cases, only one color bar is seen, representing the undivided result, *i.e.* the overall bar height still represents the mean of normalised data (n=1).

BLAST

To estimate the possible human relevance of our findings, we conducted a BLAST search of the strong hits, with the aim of investigating if bacterial strains of species known to produce the QSP, were already isolated from human samples. Therefore, amino acid sequences of the strong peptide QSM hits were blasted against the NCBI non-redundant (nr) database by Basic Local Alignment Search Tool protein (BLASTp). This blast search was performed with the organism limited to bacterial species known to produce the QSP, based on the QuorumPeps database. Only alignment hits with a 100% coverage and 100% identity were retained

In addition, for selected QSP as examples, a BLAST search was performed and bacterial strains that can synthesise the QSP as well as strains of the same species that cannot synthesise the QSP were identified. The examples of specific strains found to potentially produce specific QSP proves the probiotic applicability of our findings, i.e. providing bacterial strains that produce beneficial QSMs or do not produce harmful QSMs.

## Results

QSM effect muscle viability

The MTT assay is a widely validated and accepted viability assay for screening experiments. In this assay, the reduction of MTT into formazan, catalysed by living cell enzymes, is used as a marker of cell viability. If QSM are cytotoxic or cytostatic towards C2C12 cells, a decrease in MTT reduction is expected (viability less than 100%), while an increased C2C12 proliferation caused by QSM will result in an increased MTT reduction (viability more than 100%). The viability of the 75 QSM was between 78% (Q032) and 120% (Q192) relative to the median of all samples.

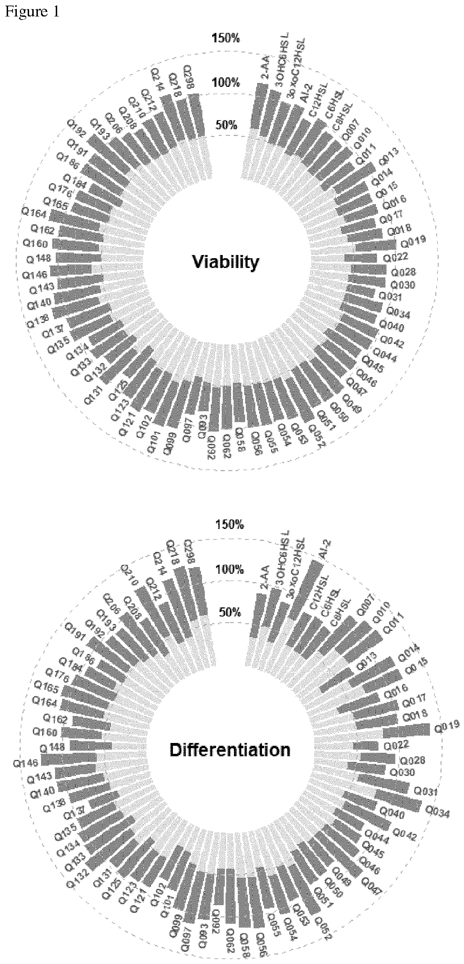

Figure 1 shows the viability and differentiation results of C2C12 myoblasts treated with QSM, using MTT assay and phase-contrast microscopy respectively. The overall bar heights represent the mean of normalised data (n=2), *i.e.* median effect of all QSM = 100%, median - 50% = 50%, median + 50% = 150%. The light grey bar represents the first result divided by two; the dark grey bar represents the second result divided by two. If the two bars are of equal length, there is no variability between the two results.

QSM influence muscle differentiation

Mimicking the *in vivo* muscle physiology, C2C12 myoblasts are able to differentiate into myotubes when they are adjacent to each other and a differentiating extracellular medium is present. Muscle wasting diseases are characterized by a decreased myotube formation. To evaluate the effects of QSM on the differentiation capacities of C2C12 myoblasts, the C2C12 cells were treated in differentiation medium with QSM at $d_0$ and $d_3$, and evaluated the myotube formation at $d_6$. At $d_6$, the staining protocol of McColl *et al.* was used to visualize the myotubes (references see above). The differentiation capacity of all QSM was between 71% (Q013) and 145% (AI-2) relative to the median of all samples (Fig. 1).

QSM modulate IL-6 secretion in muscle

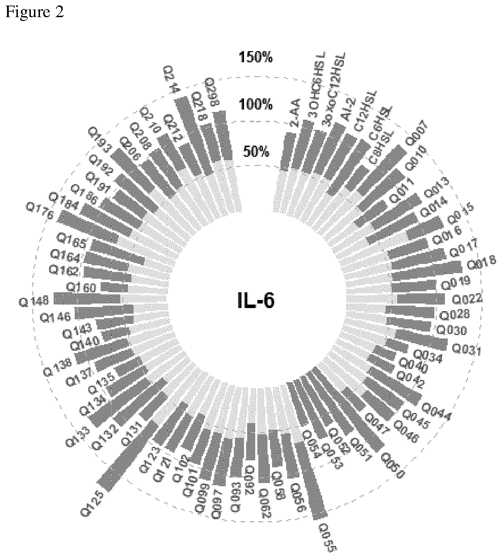

Several pro-inflammatory cytokines are elevated in sepsis, cancer, ageing and other catabolic diseases. They are thought to trigger muscle wasting (partly) through NF-kB pathways. Interleukin 6 (IL-6) is a pro-inflammatory cytokine that probably promotes activation of myoblasts and myotube regeneration at low or transiently increased levels, while sustained or strong elevated production promotes skeletal muscle wasting. In these screening experiments, the effects of QSM on myotube IL-6 production were assessed using a sandwich ELISA. Q054 and Q125 treatment showed the lowest and highest IL-6 production of all QSM, with a relative production of 51% and 171% respectively (Fig. 2).

Figure 2 shows effects of QSM on IL-6 production by C2C12 myotubes, assayed by ELISA. Overall bar heights represent the mean of normalised data (n=2), *i.e.* median effect of all QSM = 100%, median - 50% = 50%, median + 50% = 150%. The light grey bar represents the first result divided by two; the dark grey bar represents the second result divided by two. If the two bars are of equal length, there is no variability between the two results.

QSM induce muscle mitochondrial changes

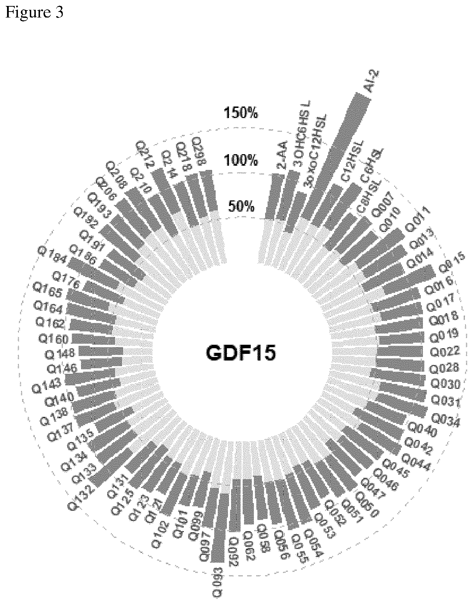

GDF15 is considered a useful biomarker for direct or indirect changes in mitochondrial function. Moreover, GDF15 is implicated in muscle wasting diseases like sarcopenia, chronic obstructive pulmonary disease cachexia and critical illness. Investigation of the 75 QSM on GDF15 secretion by C2C12 myotubes, showed effects between 80% (Q099, Q146, Q148) and 137% (Q093), with AI-2 as extreme outlier showing an effect of 216% (see Fig. 3).

Figure 3 shows the effects of QSM on GDF15 production by C2C12 myotubes, assayed by ELISA. Overall bar heights represent the mean of normalised data (n=2), *i.e.* median effect of all QSM = 100%, median - 50% = 50%, median + 50% = 150%. The light grey bar represents the first result divided by two; the dark grey bar represents the second result divided by two. If the two bars are of equal length, there is no variability between the two results.

QSM effect muscle protein degradation pathways

The net increase in muscle protein degradation compared to muscle protein synthesis plays an important role in all muscle wasting diseases, especially in the acute and subacute entities (*e.g.* critical illness and cancer cachexia). Different proteolysis pathways are involved in this increased protein degradation, with the ubiquitin-proteasome (UPS), apoptosis and autophagy systems being the most studied.

To screen the possible effects of 75 QSM on these 3 proteolysis systems, mRNA transcripts of the following representative genes were measured: atrogin and MuRF1 (UPS), caspase 3 (apoptosis system) and autophagy-related protein 7 (autophagy system).

For the sake of feasibility, a tiered approach was used: all QSM were first screened in pairs and afterwards the individual QSM of the selected paired hits were tested.

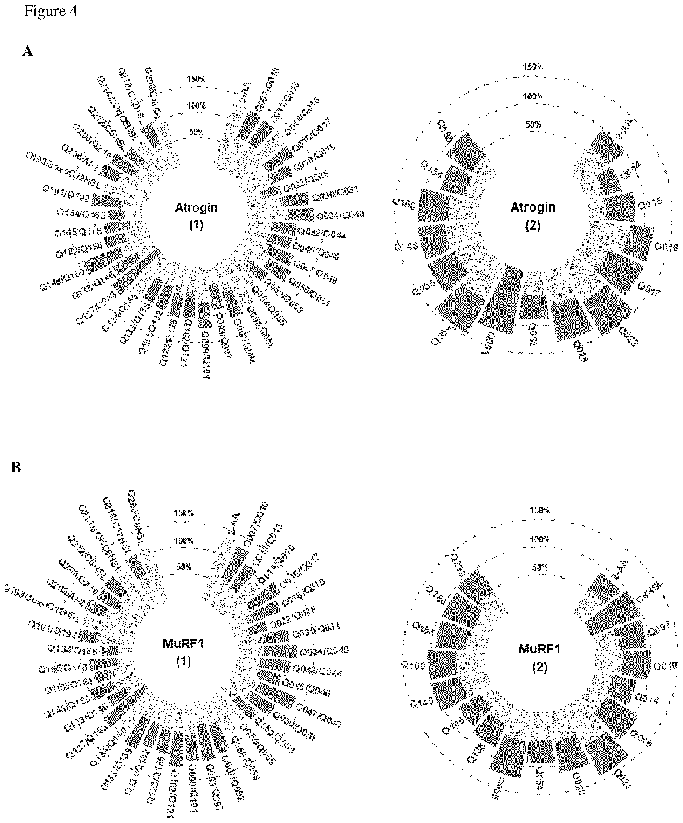

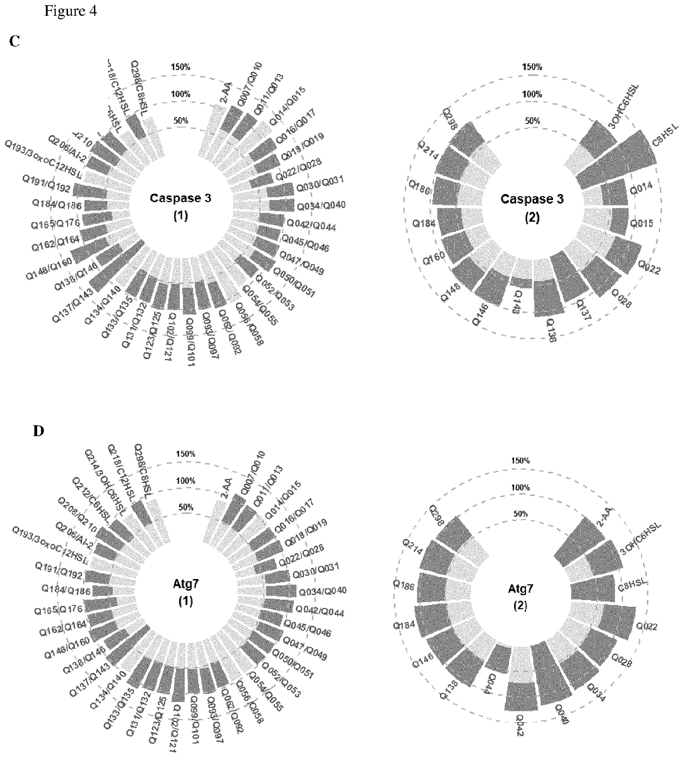

Because the individual screening is based on a selected number of QSM only, the assumption that most of the tested compounds will be inactive in a given assay cannot be taken for granted. Therefore, the normalization of this individual screening in the second phase, was performed with placebo controls instead of the median of the QSM. Also, a consistent direction of effect in both screening assays, *i.e.* the paired as well as the individual assay, was a requirement for QSM hit selection. Using this approach, 1 QSM, *i.e.* Q146, was retained as a hit, with an estimated 20% decrease in MuRF1 gene expression compared to placebo (Fig. 4).

Figure 4 shows protein degradation results of C2C12 myoblasts treated with QSM, using RT-qPCR. From top to bottom: atrogin, MuRF1, caspase 3, atg7. From left to right: paired screening (1), selected individual QSM screening (2). Overall bar heights represent the mean of normalised data (n=2), *i.e.* median effect of all QSM = 100%, median - 50% = 50%, median + 50% = 150%. The light grey bar represents the first result divided by two; the dark grey bar represents the second result divided by two. If the two bars are of equal length, there is no variability between the two results.

QSM are produced by human-relevant bacterial strains

Blasting of the strong QSP hits (Q022, Q054, Q125 and Q192) in addition to three selected hits (Q055, Q176 and Q184) showed that all, except Q192, could currently be identified in bacterial strains isolated from the human oro-gastro-intestinal or bronchial tract. Q192 was identified in bacterial strains from consumed human food. QSP-containing bacterial strains for the 7 investigated QSP are shown in Table 4 to illustrate its relevance.

**Table 4. Examples of relevant QSP-containing bacterial strains for typical QSP affecting muscle.**

| QSP | QSP-containing bacterial strain |
|-----|--------------------------------|
| Q022 | L. plantarum 43-3 (< human faeces) |
| Q054 | S. mitis SK137 (< human oral cavity) |
| Q055 | S. mitis col15 (< human sputum) |
| Q125 | L. plantarum LZ95 (< human faeces) |
| Q176 | S. mutans LCTC 10449 (< human oral cavity) |
| Q184 | E. faecalis RC73 (< human) |
| Q192 | B. subtilis B4073 (< curry soup) |

QSM are strain-specific molecules, as strains of the same species (where QSM had been previously identified) were found without QSM using Blast.

A typical relevant QSP-lacking bacterial strain for the selected exemplary QSP is shown in Table 5 to illustrate the strain specificity.

**Table 5. Relevant QSP-lacking bacterial strain for typical QSP affecting muscle.**

| QSP | QSP-lacking bacterial strain |
|-----|------------------------------|
| Q022 | L. plantarum ST-III (< kimchi food) |

(continued)

| QSP | QSP-lacking bacterial strain |
|-----|------------------------------|
| Q054 | S. mitis SK637 (< human oral cavity) |
| Q055 | S. mitis NCTC12261 (< human oral cavity) |
| Q125 | L. plantarum ZJ316 (< human faeces) |
| Q176 | S. mutans Lar01 (< human oral cavity) |
| Q184 | E. faecalis Symbioflor1 (< commercially available) |
| Q192 | B. subtilis PJ-7 (< Cheonggukjang food) |

Discussion

This study evaluated effects of QSM on C2C12 cells, with a focus on responses that are linked to muscle wasting diseases.

The set of QSM used was chosen in a way that they cover the chemical space of the currently described QSM as described in E. Wynendaele, B. Gevaert, S. Stalmans, F. Verbeke, B. De Spiegeleer, Exploring the Chemical Space of Quorum Sensing Peptides, Biopolymers 104(5) (2015) 544-551. New QSM are regularly found that make this chemical space time-dependent.

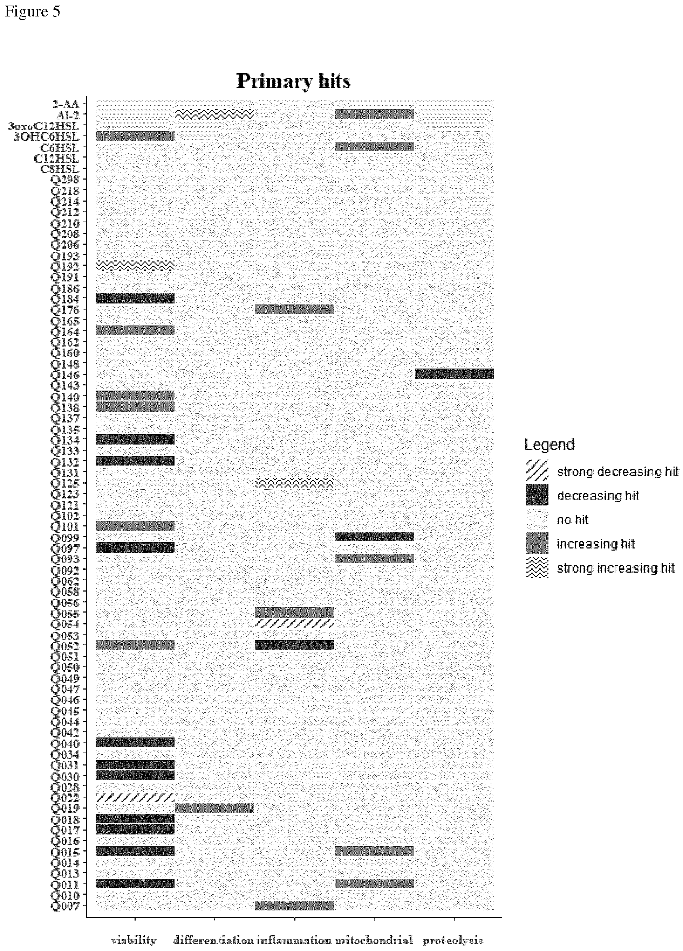

Four different approaches were used to select hits. They have in common that they aim to select QSM that are lying outside the "normal" range, *i.e.* hits are the 'extreme values'. The MAD method is similar to the well-known z-scoring; however, MAD is more robust against violation of normality. In addition to non-normality robustness, the quartile-based method is also robust against asymmetric distributions. The MAD and quartile-based methods do not take into account QSM-specific variabilities, and are therefore frequently used approaches in screenings without replicates. Because we have 2 independent biological replicates for each assay in this screening, the SSMD and robust SSMD methods that capture both the mean and QSM-specific variability are complementary to the MAD and quartile-based method. QSM that were selected using minimally 3 approaches, *i.e.* using both variability dependent and variability independent approaches, were defined as strong hits. Based on these selection criteria, 5 strong hits could be identified out of 75 QSM, while 29 QSM were selected as normal hits (Fig. 5).

Figure 5 shows overall results of QSM influencing C2C12 cells on five biological systems. Decreasing responses are indicated in dark grey or diagonal lines, while increasing responses are indicated in light grey or zig zag lines. Strong hits are defined as QSM showing an effect in minimally three hit-selection approaches.

Decreased viability and increased cell-death pathways of muscle cells are common signatures of muscle wasting diseases. The MTT assay is a validated viability assay for screening studies, wherein an assay needs to be general to minimize type 2 errors, and feasible for high throughput. Our MTT screening resulted in 7 QSM that increased C2C 12 viability and 12 QSM that decreased C2C 12 viability. The strong increasing hit Q192, together with increasing hits Q052, Q140 and Q 164 are known to be produced by *Bacillus* species, while increasing hits Q101 and Q138 are produced by *Streptococcus pneumonia* and *Lactobacillus sakei,* respectively (Table *6). Bacilli, Streptococci* and *Lactobacilli* all belong to the *Bacilli* class.

**Table 6. Overview of the active QSM with their origin and effect from in vitro experiments on C2C12 cells (strong hits in bold).**

| ID | Sequence | Origin | Effect |
|----|----------|--------|--------|
| **AI-2** | **$C_5H_{10}BO_7$** | **G+ and G-bacteria** | ↑ **differentiation** <br> ↑ GDF15 |
| 3-OH-C6-HSL | $C_{10}H_{17}NO_4$ | G- bacteria | ↑ viability |
| C6-HSL | $C_{10}H_{17}NO_3$ | G- bacteria | ↑ GDF15 |
| Q007 | YSPWTNF-NH$_2$ | *Staphylococcus epidermidis* *Staphylococcus aureus* | ↑ inflammation |

(continued)

| ID | Sequence | Origin | Effect |
|---|---|---|---|
| Q011 | AGTKPQGKPASNLVECVF SLFKKCN | *Enterococcus* | ↓ viability |
| | | *faecium* | ↑ GDF15 |
| Q015 | AKDEH | *Bacillus stearothermophilus* | ↓ viability |
| | | | ↑ GDF15 |
| Q017 | ALILTLVS | *Enterococcus faecalis* | ↓ viability |
| Q018 | ARNQT | *Bacillus subtilis* | ↓ viability |
| Q019 | NNWNN | *Escherichia coli* | ↑ differentiation |
| **Q022** | **CVGIW, thiolacton link between C1 and W5** | ***Lactobacillus plantarum*** | **↓ viability** |
| Q030 | DLRGVPNPWGWIFGR | *Streptococcus sanguis* | ↓ viability |
| Q031 | DLRNIFLKIKFKKK | *Streptococcus crista* | ↓ viability |
| Q040 | DSVCASYF, thiolacton linkage between C4 and F8 | *Staphylococcus epidermidis* | ↓ viability |
| Q052 | ERPVG | *Bacillus subtilis* | ↑ viability |
| | | | ↓ inflammation |
| **Q054** | **ESRLPKIRFDFIFPRKK** | ***Streptococcus mitis*** | **↓ inflammation** |
| Q055 | ESRVSRIILDFLFQRKK | *Streptococcus mitis* | ↑ inflammation |
| Q093 | FLVMFLSG | *Enterococcus faecalis* | ↑ GDF15 |
| Q097 | QNSPNIFGQWM, lacton link between S3 and M1 1 | *Enterococcus faecalis* | ↓ viability |
| Q099 | GKAEF | *Bacillus halodurans* | ↓ GDF15 |
| Q101 | GLWEDILYSLNIIKHNNTK GLHHPIQL | *Streptococcus pneumoniae* | ↑ viability |
| **Q125** | **KSSAYSLQMGATAIKQV KKLFKKWGW** | ***Lactobacillus plantarum*** | **↑ inflammation** |
| Q132 | LFSLVLAG | *Enterococcus faecalis* | ↓ viability |
| Q134 | LPFEF | *Bacillus cereus* | ↓ viability |
| Q138 | MAGNSSNFIHKIKQIFTHR | *Lactobacillus sakei* | ↑ viability |
| Q140 | MKAEH | *Bacillus anthracis* | ↑ viability |
| Q146 | NEVPFEF | *Bacillus cereus* | ↓ proteolysis |
| Q164 | SDLPFEH | *Bacillus cereus* | ↑ viability |
| Q176 | SGSLSTFFRLFNRSFTQAL GK | *Streptococcus mutans* | ↑ inflammation |
| Q184 | SIFTLVA | *Enterococcus faecalis* | ↓ viability |
| **Q192** | **SRNAT** | ***Bacillus subtilis*** | **↑ viability** |

Some genera of this class, *e.g. Faecalibacterium* and *Lactobacillus* have been associated with muscle wasting diseases like rheumatoid arthritis cachexia and cancer cachexia. This is consistent with our *in vitro* observations that certain QSM might be involved in these diseases. The non-peptide increasing hit 3-OH-C6-HSL is produced by different Gram-negative bacteria (*e.g. Pseudomonas*), and interestingly is already detected in human feces, with an increase seen in inflammatory bowel disease (IBD) patients. IBD is frequently associated with cachexia, making 3-OH-C6-HSL an interesting compound for diagnosis and treatment with e.g. probiotica or antagonists. Although the increased 3-OH-C6-HSL in IBD patients seems to contrast our observed increasing viability effect of 3-OH-C6-HSL in vitro, the association between cellular viability and muscle wasting is not always straightforward. For example, controlled muscle damage or death can also increase muscle mass by stimulating growth factor production, as observed during exercise.

Q022, a strong decreasing viability hit, is produced by *Lactobacillus plantarum.* A BLASTp search showed that Q022 producing *Lactobacilli plantarum* have already been isolated from human feces and saliva. Moreover, *L. plantarum* supplementation to mice increased relative muscle weight, grip strength and endurance swimming time. Although the observed decreasing viability effect of Q022 seems to contrast these mice studies, controlled muscle damage or death can also increase muscle mass by stimulating growth factor production, as observed during exercise. The other decreasing viability hits belong to *Enterococcus* (Q011, Q017, Q097, Q132, Q184), *Streptococcus* (Q030, Q031), *Staphylococcus* (Q040) or *Bacillus* (Q015, Q018, Q134) species. *Enterococci, Streptococci* and *Staphylococci* are common causative organisms in sepsis, a frequently encountered trigger for acute myopathy. Our *in vitro* viability results are thus consistent with these clinical observations, providing evidence that certain QSM play a role in muscle wasting diseases.

In both *in vitro* and *in vivo* muscle wasting models, decreased differentiation of mononuclear myocytes into polynuclear myotubes is observed, resulting in a diminished force. C2C12 cells treated with AI-2 and Q019 both showed an increased myotube formation, with AI-2 being a strong hit. AI-2 is produced and recognized by a large number of Gram-positive and Gram-negative genera, and has the potential to shift the abundances of major phyla in mammalian gut microbiota. The data show that AI-2 (or AI-2 mimics) acts on muscle cells and can be protective against muscle wasting diseases. Q019 is produced by the Gram-negative *Escherichia coli* (*E. coli*), in contrast to most other quorum sensing peptides that are produced by Gram-positive genera. It acts as an extracellular death factor, inducing death of stressed subpopulations of *E. coli,* thereby permitting the survival of the bacterial population as a whole. *E. coli* belongs to the family of *Enterobacteriaceae,* which are known to be increased in the gut microbiota of frail older people and are also frequent causative organisms in sepsis. Our screening results thus show that Q019 is an interesting compound to use in the context of muscle wasting diseases.

Inflammation is evident in acute and subacute muscle wasting diseases. Also in chronic muscle wasting diseases, most studies suggest an increased low-grade inflammation as contributor to the disease. Q007, Q055, Q125 and Q176 increased IL-6 production in C2C12 cells (pro-inflammatory), while Q052 and Q054 decreased the IL-6 production (anti-inflammatory). The known producers of these six QSM belong to the genera of *Staphylococcus, Streptococcus, Lactobacillus* and *Bacillus.* Of these six IL-6 influencing QSM, Q125 and Q054 were identified as strong hits. A BLASTp search of Q054 and Q125 showed that both of them were already found in bacteria isolated from the human gastro-intestinal tract. Q052 (*Bacillus subtilis*), an anti-inflammatory hit, was also identified as an increasing viability hit, confirming its putative effects on muscle cells.

AI-2, Q011, Q015, Q093 and C6-HSL treatment increased GDF15 production in C2C12 myotubes, while Q099 decreased the GDF15 production (Fig. 5 and Table 6). Interesting is that AI-2, Q011 and Q015 were also identified as viability QSM in our findings, with AI-2 increasing both C2C12 viability and GDF15 production. Skeletal muscle mitochondrial function is a critical determinant of both muscle and whole body homeostatis. Hence, a disturbed mitochondrial function is often part of the pathophysiology of muscle wasting diseases. In this study, GDF15 was chosen as biomarker for mitochondrial disturbations, as it is produced in response to direct and indirect mitochondrial stress, independent of the nature of the stress. Examples of indirect mitochondrial stress and GDF15 secretion are oxidative stress and endoplasmic reticulum (ER) stress. Studies showing that GDF15 levels are increased in muscle wasting diseases confirm the utility of GDF15 as biomarker of mitochondrial changes in these diseases.

None of the tested QSM induced the expression of protein degradation genes. This could i.a. suggest that for example 1) the QSM affect only enzyme activity and not mRNA expression of the tested genes, 2) the QSM induce muscle wasting using other pathways than the ones examined here, or 3) the expression of the tested genes has changed at other QSM concentrations or time-points than 24h after QSM incubation.

Q146, also called PapRIII and produced by *Bacillus cereus,* decreased the expression of MuRF1 with 20% in C2C12 cells. As MuRF1 expression is increased in most muscle wasting diseases, our results show a protective effect of Q146 on muscle cells.

Overall, this study shows a role of QSM in the gut-muscle axis. The diversity of QSM, biological responses and hit identification approaches allow to cover a large area of the QSM-muscle space.

## QSP analytics in bacterial strains

The QSM produced by specific bacterial strains can be analyzed by qPCR and/or UPLC/MS as described above. The found QSM is compared to the knowledge base for QSM's and the strain is qualified as positive, neutral or negative in relation to muscle wasting.

The table shows examples of relevant bacterial strains where the bacterial strain is a wild-type strain that does not produce a functional QSMs of the invention identified to have a negative impact on muscle homeostasis and/or to induce muscle wasting. We evaluated on both DNA level (qPCR) and peptide level (UPLC/MS). Other strains (not shown) from the same species were found to be positive on qPCR and/or UPLC/MS.

**Examples of relevant QSP-lacking bacterial strains for typical QSP negatively affecting muscle.**

| QSP | Species | Strain | qPCR (DNA) | UPLC/MS (peptide) |
|---|---|---|---|---|
| Q055 | *Streptococcus mitis* | LMG 14557 | - | - |
| | | LMG 14554 | - | - |
| | | LMG 14555 | - | - |
| | | LMG 14552 | - | - |
| Q125 | *Lactobacillus plantarum* | LP02 (< Symbiosis Defencia) | - | - |
| | | W21 (< CellCare Probiotica) | - | - |
| | | LMG 26367 (Flora+Turbo) | - | - |
| | | LMG 9211 | - | - |
| | | LMG 26655 (< Golden Naturals Crandophilus) | - | - |
| | | LMG 26655 (< Golden Naturals Probiotica) | - | - |
| | | L. plantarum strain from PUUR Probiotic | - | - |
| | | W21 (< New Care Bifido Lacto complex) | - | - |
| | | L. plantarum strain from Vitotaal Pro-bioticum | - | - |
| Q184 | *Enterococcus faecalis* | LMG 8222 | - | - |
| | | LMG 7937 | - | - |
| | | LMG 20724 | - | - |
| | | Symbioflor 1 | - | - |

## Dose-response experiments

QSPs show dose-response effect in C2C12 cells

In a further set of experiments, using similar methods as described for the screening experiments, a number of QSPs were tested for a dose response relation in C2C12 cells. Typical curves were obtained for relative IL6 production, for Q55, Q125 and Q176. Results are given in Tables 7.1, 7.2 and 7.3, with the EC10 values under each table.

**Table 7.1. Q176 concentration dependent IL-6 response of C2C12 myotubes.**

| Concentration ($\mu$M) | N | Relative IL-6 | SEM |
|---|---|---|---|
| 0 | 38 | 0.98 | 0.03 |
| 0.20 | 10 | 1.00 | 0.06 |
| 0.39 | 10 | 0.89 | 0.04 |
| 0.78 | 10 | 0.98 | 0.07 |
| 1.56 | 10 | 1.53 | 0.09 |
| 3.13 | 10 | 1.61 | 0.16 |
| 6.25 | 10 | 2.39 | 0.23 |
| 12.5 | 5 | 3.24 | 0.26 |
| 25 | 5 | 3.49 | 0.39 |

(continued)

| Concentration (μM) | N | Relative IL-6 | SEM |
|---|---|---|---|
| 50 | 5 | 5.81 | 0.44 |
| 100 | 5 | 6.01 | 0.56 |
| EC10 = 2.8 μM (95% CI = 1.7 μM; 3.9 μM) | | | |

**Table 7.2. Q055 concentration dependent IL-6 response of C2C12 mytubes.**

| Concentration (μM) | N | Relative IL-6 | SEM |
|---|---|---|---|
| 0 | 36 | 1.03 | 0.04 |
| 0.20 | 10 | 0.94 | 0.07 |
| 0.39 | 10 | 1.07 | 0.08 |
| 0.78 | 10 | 1.05 | 0.08 |
| 1.56 | 10 | 0.93 | 0.08 |
| 3.13 | 10 | 1.24 | 0.05 |
| 6.25 | 10 | 1.24 | 0.08 |
| 12.5 | 5 | 3.60 | 0.11 |
| 25 | 4 | 5.56 | 0.50 |
| 50 | 5 | 8.94 | 0.97 |
| 100 | 5 | 6.48 | 0.59 |
| EC10 = 7.4 μM (95% CI = 5.9 μM; 8.9 μM) | | | |

**Table 7.3. Q125 concentration dependent IL-6 response of C2C12 myotubes.**

| Concentration (μM) | N | Relative IL-6 | SEM |
|---|---|---|---|
| 0 | 37 | 1.02 | 0.02 |
| 0.20 | 10 | 0.89 | 0.05 |
| 0.39 | 10 | 0.90 | 0.04 |
| 0.78 | 10 | 1.15 | 0.06 |
| 1.56 | 10 | 1.28 | 0.09 |
| 3.13 | 10 | 1.96 | 0.13 |
| 6.25 | 10 | 2.08 | 0.17 |
| 12.5 | 5 | 4.89 | 0.45 |
| 25 | 5 | 5.55 | 0.44 |
| 50 | 5 | 8.23 | 0.53 |
| 100 | 5 | 9.80 | 0.50 |
| EC10 = 4.3 μM (95% CI = 3.5 μM; 5.1 μM) | | | |

**Table 7.4. Q022 concentration dependent MTT response in C2C12 myotubes.**

| Concentration (μM) | N | Relative MTT | SEM |
|---|---|---|---|
| 0 | 9 | 1.00 | 0.03 |

(continued)

| Concentration (μM) | N | Relative MTT | SEM |
|---|---|---|---|
| 0.10 | 5 | 0.99 | 0.02 |
| 0.50 | 5 | 1.02 | 0.03 |
| 1.00 | 5 | 1.03 | 0.03 |
| 5.00 | 5 | 0.86 | 0.03 |
| 10 | 5 | 0.73 | 0.03 |
| 50 | 5 | 0.58 | 0.03 |
| 100 | 5 | 0.55 | 0.02 |
| IC10 = 2.4 μM (95% CI = 0.8 μM; 3.9 μM) | | | |

**Table 7.5. 0184 concentration dependent MTT response in C2C12 mvotubes.**

| Concentration (μM) | N | Relative MTT | SEM |
|---|---|---|---|
| 0 | 24 | 1.00 | 0.02 |
| 0.10 | 5 | 0.68 | 0.02 |
| 0.50 | 5 | 0.60 | 0.02 |
| 1.00 | 5 | 0.56 | 0.02 |
| 5.00 | 5 | 0.49 | 0.01 |
| 10 | 5 | 0.49 | 0.02 |
| IC10 = 0.01 μM (95% CI= 0.00 μM; 0.02 μM) | | | |

These results indicate that selected bacterial quorum sensing molecules have a concentration-dependent inflammatory influence on muscle cells, and can be used for diagnostic and prognosis purposes (as biomarkers), as well as in therapeutic strategies.

In a further set of experiments, the dose response effect of QSPs Q22 and Q184 was assessed on MTT reduction of C2C12 myotubes (tables 7.4 and 7.5). Effects of the QSPs were also evaluated on different stages of muscle cell formation, and effects are observed in both early stages (myoblasts) as in the more mature forms (myotubes), which can differ in quantitative terms as observed for example in the MTT reduction after treatment with Q022 and Q184. Results are given in the table 8.

**Table 8: Characteristics of the example concentration response curves in C2C12 myoblasts as well as myotubes.**

| | Batch | Slope | $I_{max}$ (relative MTT) | $IC_{10}$ (μM) | $IC_{50}$ (μM) |
|---|---|---|---|---|---|
| **Q022** | Blasts | 1.26 | 0.63 | 0.76 | 4.33 |
| | Tubes | 1.90 | 0.55 | 2.38 | 7.55 |
| **Q184** | Blasts | 1.67 | 0.63 | 0.40 | 1.49 |
| | Tubes | 0.68 | 0.48 | 0.01 | 0.06 |

QSPs show similar dose-response effects in human muscle cells as in the murine

C2C12 cells, indicating the translational relevance.

Immortalised human myoblast cell lines were cultured at 37°C in a 5% $CO_2$ humidified incubator in PromoCell Skeletal Muscle Cell Growth Medium supplemented with 20% fetal bovine serum, insulin (10 μg/mL), dexamethasone (0.4 μg/mL), fetuin (50 μg/mL), hEGF (10 ng/mL), bFGF (1 ng/mL), penicillin (100 units/ml), and streptomycin (100

μg/ml). The medium was renewed each 2-3 days and confluency was kept below 80%. For differentiation of the human myoblasts into myotubes, proliferation medium was replaced by differentiation medium when cells reached 80% confluency. Differentiation medium consisted of high glucose Glutamax DMEM supplemented with penicillin (100 units/ml), streptomycin (100 μg/ml) and insulin (10 μg/mL).

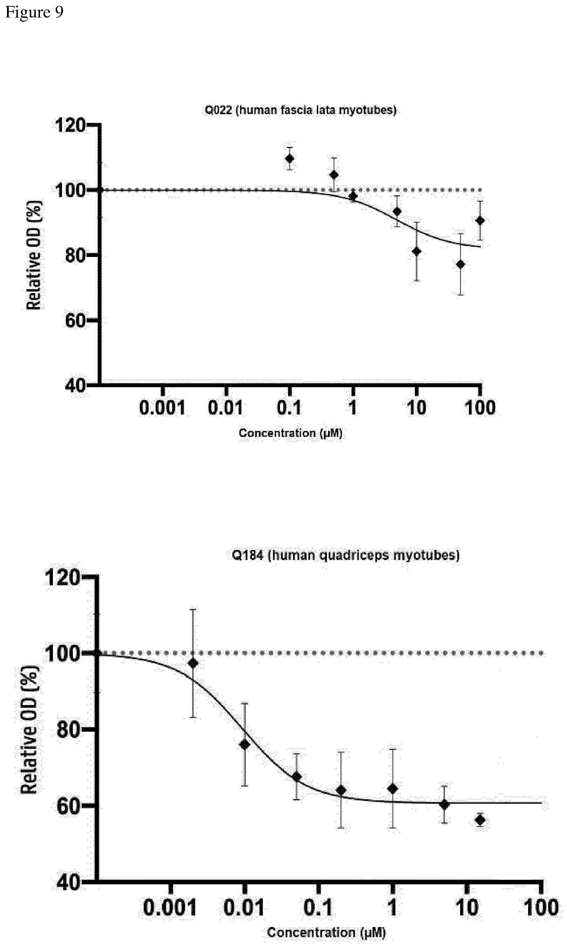

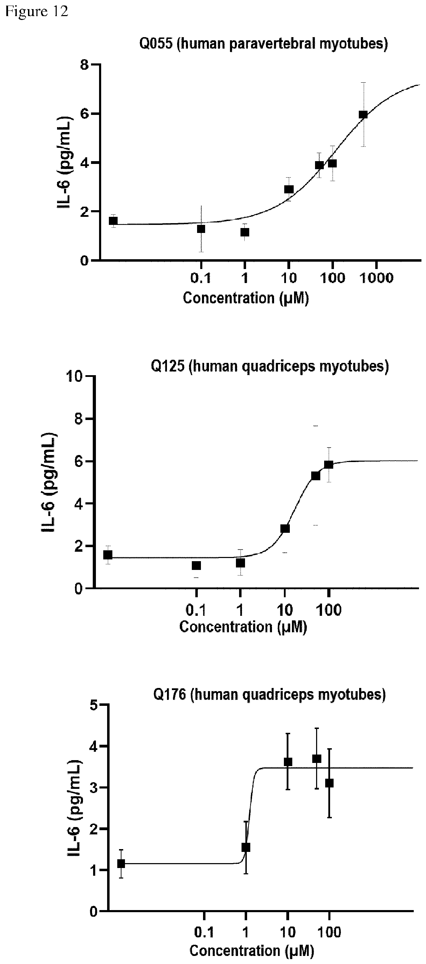

Using similar methods as described for the C2C12 experiments, a number of QSPs were tested for a dose response relation in human muscle cells. Typical curves were obtained for MTT reduction for Q022 and Q184 (Figure 9) and IL6 production for Q055, Q125 and Q176 (Figure 12).

Such dose response in both murine and human muscle cells is further evidence of significant biological effects.

## Agonist and antagonist experiments

### Alanine scans modulate the biological activity

The activity on muscle cells of some QSPs was modulated by alanine-scans. The results show the importance of certain amino acid residues for the biological activity on muscle cells.

Changing amino acids is a flexible way of increasing as well as of decreasing the activity, as demonstrated by typical examples hereafter.

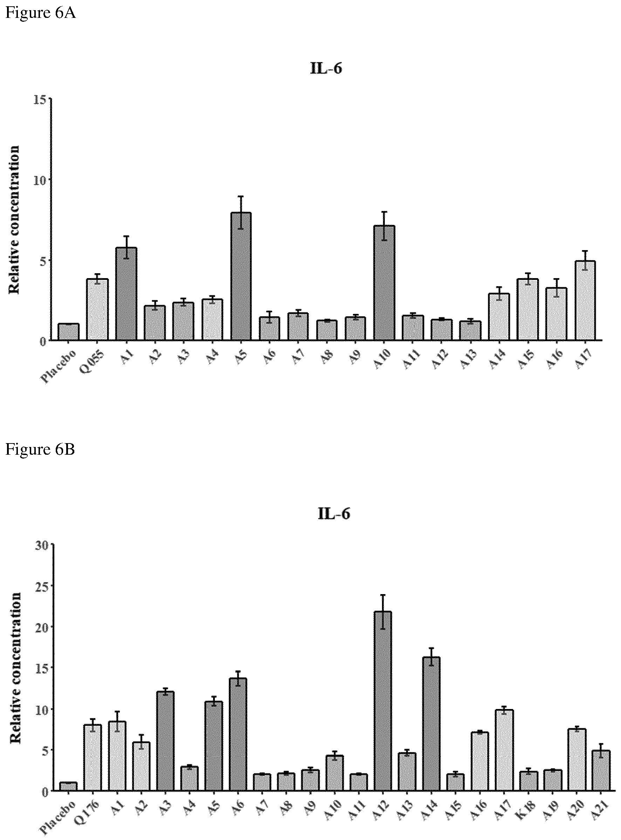

Fig 6A and 6B respectively show the relative IL-6 secretion of Q055 (Fig 6A) and Q176 (Fig B) ala-scan peptides (with placebo = 1). Means ± SEM, n = 6 (for placebo n = 76). Bars in dark grey are statistically different from Q055 respectively Q176 (p < 0.05, Dunnett's t-test, 2-sided).

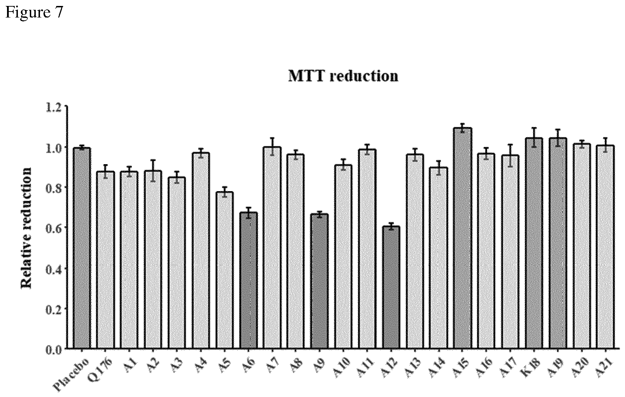

Fig. 7 shows the relative MTT reduction of Q176 ala-scan peptides (with placebo set to 1). Means ± SEM, n = 6 (for placebo n = 76). Bars in dark grey are statistically different from Q176 (p < 0.05, Dunnett's t-test, 2-sided).

### Antagonist or Agonist for QSPs

This example shows the possibility of designing peptides or peptidederivatives with an antagonistic activity (antagonist or partial agonist). Co-treating C2C12 cells with Q055 together with one of its ala-scan derivatives (Q055-A6 SRVSAIILDFLFQRKK (SEQ ID NO: 77) inhibited the Q055 mediated IL-6 induction. A further derivate ESRVSRIIADFLFQRKK (SEQ ID NO: 78; QSP055-A9) was shown to possess an antagonistic effect.

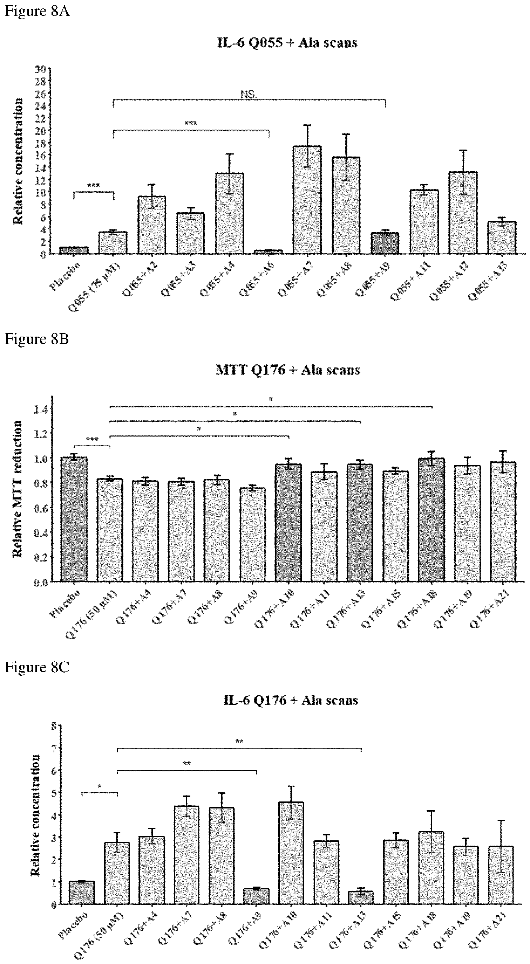

Fig. 8A shows the IL-6 response of co-treatments of Q055 (75 μM) and derivatives (alascan-peptides) as possible antagonists (90 μM), relative to placebo (placebo = 1) or as agonists. Means ± SEM, n = 6. * P < 0.05; ** P < 0.01, *** P < 0.001; paired Student's t test.

Fig 8B shows the MTT response of co-treatments of Q176 (50 μM) and derivatives (alascan-peptides) as possible antagonists (60 μM), relative to placebo (placebo = 1). Means ± SEM, n = 6. * P < 0.05; ** P < 0.01, *** P < 0.001; paired Student's t test.

Fig 8C shows the IL-6 response of co-treatments of Q176 (50 μM) and derivatives (alascan-peptides) as possible antagonists (60 μM), relative to placebo (placebo = 1). Means ± SEM, n = 6. * P < 0.05; ** P < 0.01, *** P < 0.001; paired Student's t test.

Co-treating C2C12 cells with Q176 together with its derived Ala-scans identifies 2 peptides (Q176-A9: GSLSTFFALFNRSFTQALGK (SEQ ID NO: 79) and Q176-A13; GSLSTFFRLFNASFTQALGK (SEQ ID NO:80) as inhibiting the Q176 mediated IL-6 induction, and 3 peptides (Q176-A10, Q176-A13 and Q176-A18) as inhibiting the Q176 mediated MTT effect. The presence of QSP176-A13 clearly abolishes both effects observed with the unmodified QSP176 alone.

The invention therefore also resides in an agonist or antagonist, wherein the agonist or antagonist is an oligopeptide, homologous to the QSP.

## In vivo experiments

### QSPs influence muscle wasting in *C. elegans* model

The in-vivo relevance of QSPs in modulating the muscle functionality is demonstrated in an in-vivo *C. elegans* model, extending the in-vitro conclusions to the in-vivo situation. The *C. elegans* model is a well-established aging model: they diminish in locomotory vigor as they age, a phenomenon that is attributed to a physical deterioration of muscle and synapses. QSPs were introduced in the medium of living *C.elegans.* As comparisons, a placebo experiment was used as negative control, while DMSO was used as positive control.

Using automatic, objective imaging software, different swimming behaviour variables associated with this decrease in locomotory vigor or muscle wasting were investigated. Wave initiation rate, travel speed, brush stroke and activity index do decrease with muscle wasting, while body wave number, asymmetry, curling and stretch do increase with muscle wasting, as described in Restif et al., Plos Computational Biology, 2014.

Results of Q184 are given in the following tables 9 and 10, demonstrating the good correlation with the in-vitro results.

More in particular, the results in these tables show Q184-associated changes in three swimming parameters in wild-type adults at day 3 (Table 9) and day 12 (Table 10) (n=120-170 from 3 independent trials for each condition). Data presented are means $\pm$ s.e.m (placebo set to 1.0).

**Table 9.1. Wave initiation rate of C. elegans at day 3.**

| Condition | N | Wave initiation rate | SEM |
|---|---|---|---|
| DMSO | 121 | 0.58 | 0.04 |
| Placebo | 155 | 1.00 | 0.03 |
| Q184 (1 $\mu$M) | 126 | 0.90 | 0.03 |

**Table 9.2. Body wave number of C. elegans at day 3.**

| Condition | N | Body wave number | SEM |
|---|---|---|---|
| DMSO | 121 | 2.04 | 0.10 |
| Placebo | 155 | 1.00 | 0.05 |
| Q184 (1 $\mu$M) | 126 | 1.01 | 0.06 |

**Table 9.3. Asymmetry of C. elegans at day 3.**

| Condition | N | Asymmetry | SEM |
|---|---|---|---|
| DMSO | 121 | 3.11 | 0.38 |
| Placebo | 155 | 1.00 | 0.13 |
| Q184 (1 $\mu$M) | 126 | 1.10 | 0.12 |

**Table 9.4. Stretch of C. elegans at day 3.**

| Condition | N | Stretch | SEM |
|---|---|---|---|
| DMSO | 121 | 1.10 | 0.02 |
| Placebo | 155 | 1.00 | 0.01 |
| Q184 (1 $\mu$M) | 126 | 1.03 | 0.02 |

**Table 9.5. Curling of C. elegans at day 3.**

| Condition | N | Curling | SEM |
|---|---|---|---|
| DMSO | 121 | 1.55 | 0.29 |
| Placebo | 155 | 1.00 | 0.20 |
| Q184 (1 $\mu$M) | 126 | 0.92 | 0.14 |

**Table 9.6. Travel speed of C. elegans at day 3.**

| Condition | N | Travel speed | SEM |
|---|---|---|---|
| DMSO | 121 | 0.85 | 0.08 |
| Placebo | 155 | 1.00 | 0.08 |

(continued)

| Condition | N | Travel speed | SEM |
|---|---|---|---|
| Q184 (1 μM) | 126 | 0.55 | 0.04 |

**Table 9.7. Brush stroke of C. eleeans at day 3.**

| Condition | N | Brush stroke | SEM |
|---|---|---|---|
| DMSO | 121 | 0.68 | 0.06 |
| Placebo | 155 | 1.00 | 0.04 |
| Q184 (1 μM) | 126 | 0.87 | 0.05 |

**Table 9.8. Activity index of C. elegans at day 3.**

| Condition | N | Activity index | SEM |
|---|---|---|---|
| DMSO | 120 | 0.60 | 0.05 |
| Placebo | 148 | 1.00 | 0.04 |
| Q184 (1 μM) | 119 | 0.82 | 0.05 |

**Table 10.1. Wave initiation rate of C. elegans at day 12.**

| Condition | N | Wave initiation rate | SEM |
|---|---|---|---|
| DMSO | 100 | 0.39 | 0.04 |
| Placebo | 190 | 1.00 | 0.03 |
| Q184 (1 μM) | 137 | 0.84 | 0.04 |

**Table 10.2. Body wave number of C. elegans at day 12.**

| Condition | N | Body wave number | SEM |
|---|---|---|---|
| DMSO | 100 | 2.59 | 0.10 |
| Placebo | 190 | 1.00 | 0.06 |
| Q184 (1 μM) | 137 | 1.29 | 0.09 |

**Table 10.3. Asymmetry of C. elegans at day 12.**

| Condition | N | Asymmetry | SEM |
|---|---|---|---|
| DMSO | 100 | 2.50 | 0.26 |
| Placebo | 190 | 1.00 | 0.09 |
| Q184 (1 μM) | 137 | 1.37 | 0.15 |

**Table 10.4. Stretch of C. elegans at day 12.**

| Condition | N | Stretch | SEM |
|---|---|---|---|
| DMSO | 100 | 0.91 | 0.01 |

(continued)

| Condition | N | Stretch | SEM |
|---|---|---|---|
| Placebo | 190 | 1.00 | 0.01 |
| Q184 (1 $\mu$M) | 137 | 1.06 | 0.02 |

**Table 10.5. Curling of C. elegans at day 12.**

| Condition | N | Curling | SEM |
|---|---|---|---|
| DMSO | 100 | 0.08 | 0.04 |
| Placebo | 190 | 1.00 | 0.17 |
| Q184 (1 $\mu$M) | 137 | 1.50 | 0.37 |

**Table 10.6. Travel speed of C. elegans at day 12.**

| Condition | N | Travel speed | SEM |
|---|---|---|---|
| DMSO | 100 | 0.49 | 0.06 |
| Placebo | 190 | 1.00 | 0.05 |
| Q184 (1 $\mu$M) | 137 | 0.81 | 0.06 |

**Table 10.7. Brush stroke of C. elegans at day 12.**

| Condition | N | Brush stroke | SEM |
|---|---|---|---|
| DMSO | 100 | 0.48 | 0.03 |
| Placebo | 190 | 1.00 | 0.04 |
| Q184 (1 $\mu$M) | 137 | 0.89 | 0.05 |

**Table 10.8. Activity index of C. elegans at day 12.**

| Condition | N | Activity index | SEM |
|---|---|---|---|
| DMSO | 100 | 0.31 | 0.03 |
| Placebo | 189 | 1.00 | 0.04 |
| Q184 (1 $\mu$M) | 134 | 0.85 | 0.05 |

These result show that 1 $\mu$M of Q184 induced a muscle wasting phenotype in *C. elegans.* This effect was seen both at day 3 and day 12 (independent experiments). At day 3, a significant decrease in wave initiation rate, travel speed, brush stroke and activity index was observed. After 12 days of Q184 incubation, a significant decrease in wave initiation rate, travel speed, brush stroke and activity index was observed, and a significant increase in body wave number, asymmetry and stretch. Curling, a variable expected to increase with muscle wasting/ageing, did also increase after 12 days Q184 1 $\mu$M treatment.

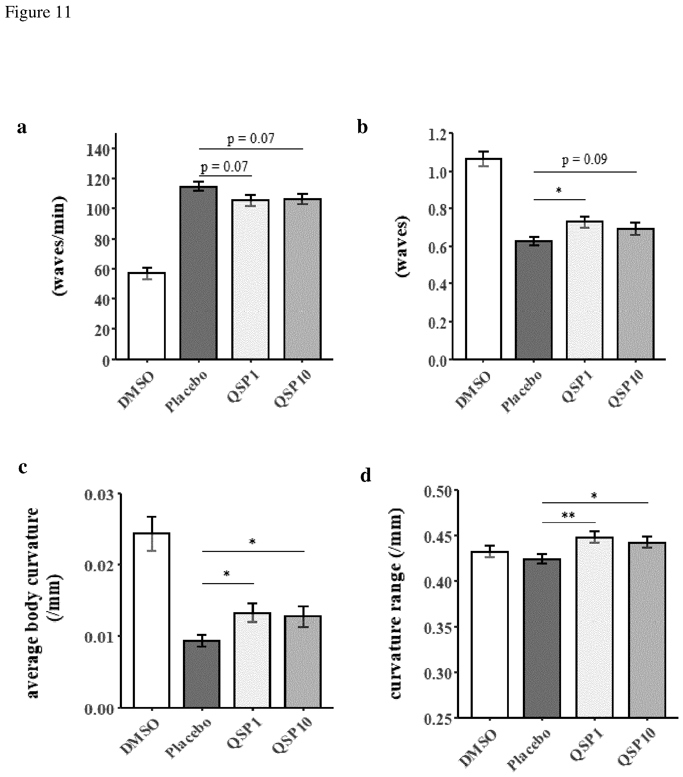

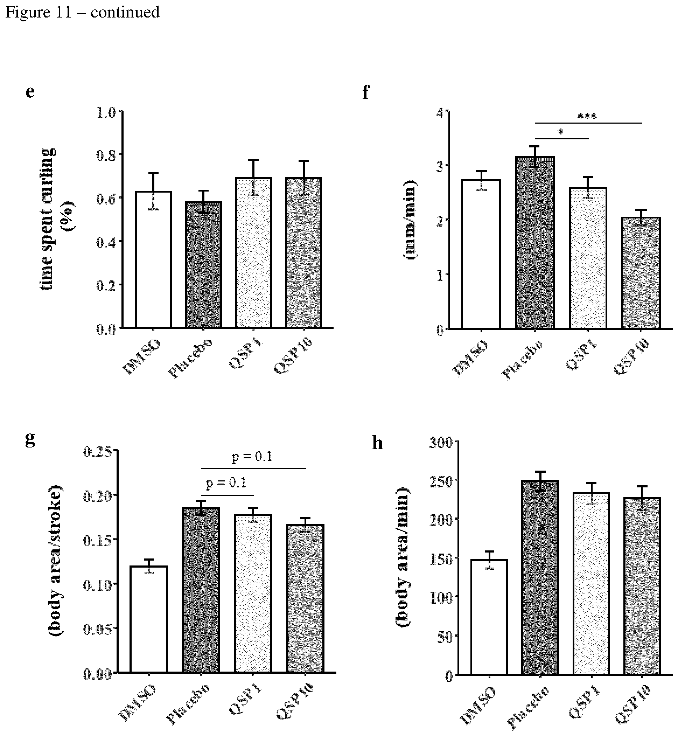

In further sets of *C. elegans* experiments, the muscle wasting effects of Q184 and Q022 in vivo were confirmed (Figure 10 and Figure 11).

Altogether, these in-vivo data collected at 2 different time-points in *C. elegans* highlight the in vivo confirmation of the in vitro obtained results of the QSP, for example Q022 and Q184 reducing the metabolic activity in muscle both in vitro and in vivo, inducing a muscle wasting phenotype.

The concentration of 1 $\mu$M for the in vivo *C. elegans* experiments is assumed to be physiologically relevant. QSM concentrations in human and mice plasma are estimated between 0.1 and 1 nM, while concentrations in pre-epithelium barrier matrices (saliva, faeces, sputum) are estimated to be in the size order of 0.1 to 1 $\mu$M. See, e.g. F.

Verbeke, S. De Craemer, N. Debunne, Y. Janssens, E. Wynendaele, C. Van de Wiele, B. De Spiegeleer, Peptides as Quorum Sensing Molecules:Measurement Techniques and Obtained Levels In vitro and In vivo, Frontiers in Neuroscience 11 (2017) 1-18.

Because it is expected that these concentrations in vivo are present for a long time, e.g. months or years in the context of cachexia or sarcopenia, higher concentrations than 0.1 μM should be tested when incubating the worms with QSM for short term, with the aim of reaching similar total exposure. As an example, if Q184 is added to C. elegans at 1 μM during 12 days (it was confirmed that Q184 has a high stability in the medium), 12 μM.d is the total exposure. This would correspond to a total duration of 4 months (120 days) at a faecal expected QSM concentration of 100 nM. Also if adjusting for the short lifespan of C. elegans vs. humans, a concentration of 1 μM is still physiologically relevant (knowing that QSM concentrations up to 1 μM are measured in humans in vivo pre-epithelium).

QSMs influence muscle wasting in murine model

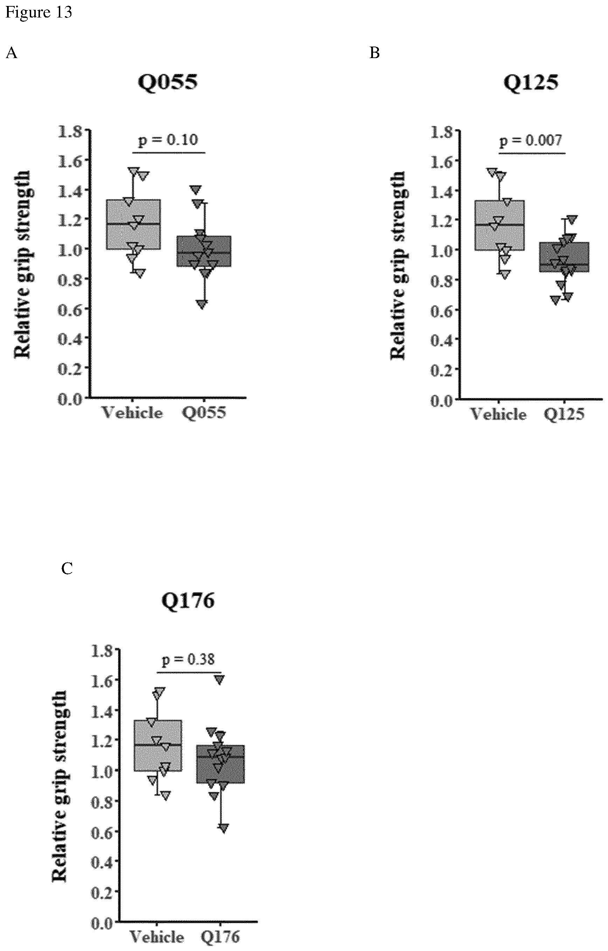

The in-vivo relevance of QSMs in modulating the muscle functionality is further demonstrated in an in-vivo murine model, extending the previous in-vitro and in-vivo conclusions. All experimental procedures were performed in accordance with institutional guidelines for animal studies and were approved by an ethics committee (ECD 19-17, University Hospital Ghent). C57Bl6/J WT mice aged 12 months were housed conventionally in a constant temperature (20-22 °C) and humidity (50-60%) animal room, with a 12 h-12h light-dark cycle and free access to food and water. Mice were daily i.p. injected with 100 μL QSP at 0.5 μmol/kg/day or with an equal volume of PBS (placebo-control). Investigators were blinded to the group allocation during the protocol. After 2 weeks of daily injection, mice were allowed to grasp a horizontal grid connected to a dynamometer with all four limbs and were pulled backwards three times. The force applied to the grid each time before the animal lost its grip was recorded in grams. The average of the three measures was normalized to the whole body weight of each mouse. All grip-values were measured by the same researcher, blinded for the treatment. Figure 13 shows the results hereof, demonstrating the in vivo confirmation of the in vitro obtained results of the QSP, for example Q055, Q125 and Q176 inducing a muscle wasting phenotype with in vitro inflammation and in vivo muscle strength decrease.

**Claims**

1. Diagnostic in vitro method for analyzing the presence of a quorum sensing molecule (QSM) in a sample of a mammal to assess the presence of at least one QSM that influences muscle wasting and wherein the QSM is selected from the group consisting of: CVGIW (SEQ ID NO: 11; QSP022), ESRVSRIILDFLFQRKK (SEQ ID NO: 28; QSP055), KSSAYSLQMGATAIKQVKKLFKKWGW (SEQ ID NO: 40; QSP0125), SGSLSTFFRLFNRSFTQALGK (SEQ ID NO: 56; QSP176), and SIFTLVA (SEQ ID NO: 57; QSP184).

2. Diagnostic method according to claim 1, wherein the method is done on feces and/or blood and/or saliva and/or skin-swabs, and/or other body-fluids, and/or other samples of the mammal.

3. Diagnostic method according to any one of claims 1-2, wherein the mammal is a human, companion animal or farm animal.

4. Diagnostic method according to any one of claims 1-3, wherein the presence of at least one QSM is indicating a risk of or the presence of muscle wasting.

5. Bacterial strain from the species *Lactobacillus plantarum, Enterococcus faecalis, Streptococcus mitis* or *Streptococcus mutans,* wherein said strain does not produce one or more of the QSMs selected from the group consisting of: CVGIW (SEQ ID NO: 11; QSP022), ESRVSRIILDFLFQRKK (SEQ ID NO: 28; QSP055), KSSAYSLQMGATAIKQVKKLFKKWGW (SEQ ID NO: 40; QSP0125), SGSLSTFFRLFNRSFTQALGK (SEQ ID NO: 56; QSP176), and SIFTLVA (SEQ ID NO: 57; QSP184), for use in preventing, treating or reducing muscle wasting associated with muscular dystrophies, amyotrophic lateral sclerosis, inflammatory myopathies, denervation muscle atrophies, neurological disease, cachexia, anorexia-cachexia syndrome, cancers, rheumatoid arthritis, osteoarthritis, diabetes, inflammatory bowel disease, liver cirrhosis, chronic obstructive pulmonary disease, pulmonary fibrosis, chronic renal disease, chronic heart failure, sarcopenia, physical frailty, androgen deprivation, corticosteroid myopathy, trauma (e.g. burns), critical illness myopathy (e.g. sepsis), acquired immune deficiency syndrome (AIDS) or cardiomyopathy.

6. Bacterial strain for use according to claim 5, wherein said strain replaces or reduces in the gut the strains that have QSMs shown to negatively influence aspects of muscle homeostasis.

**7.** Bacterial strain for use according to claim 6, wherein the strains that have QSMs shown to negatively influence aspects of muscle homeostasis are bacterial strains that produce one or more of the QSMs selected from the group consisting of: CVGIW (SEQ ID NO: 11; QSP022), ESRVSRIILDFLFQRKK (SEQ ID NO: 28; QSP055), KSSAYSLQMGATAIKQVKKLFKKWGW (SEQ ID NO: 40; QSP0125), SGSLSTFFRLFNRSFTQALGK (SEQ ID NO: 56; QSP176), and SIFTLVA (SEQ ID NO: 57; QSP184).

**8.** Bacterial strain for use according to any one of claims 5-7, wherein the bacterial strain is part of a composition, in particular a live biotherapeutic product or probioticum.

**9.** Method of screening bacterial strains suitable as live biotherapeutic product or probiotic strain, wherein the QSM produced by said bacterial strain is analysed, and wherein the absence of one or more of the QSMs selected from the group consisting of: CVGIW (SEQ ID NO: 11; QSP022), ESRVSRIILDFLFQRKK (SEQ ID NO: 28; QSP055), KSSAYSLQMGATAIKQVKKLFKKWGW (SEQ ID NO: 40; QSP0125), SGSLSTFFRLFNRSFTQALGK (SEQ ID NO: 56; QSP176), and SIFTLVA (SEQ ID NO: 57; QSP184) is indicative of a strain that is suitable as live biotherapeutic product or probiotic.

**10.** Peptide antagonist of one or more of the peptides selected from the group consisting of: ESRVSRIILDFLFQRKK (SEQ ID NO: 28; QSP055), and SGSLSTFFRLFNRSFTQALGK (SEQ ID NO: 56; QSP176), wherein the peptide antagonist of QSP055 (SEQ ID NO: 28) is a peptide having the amino acid sequence SRVSAIILDFLFQRKK (SEQ ID NO: 77; QSP055-A6) or the amino acid sequence ESRVSRIIADFLFQRKK (SEQ ID NO: 78; QSP055-A9), and wherein the peptide antagonist of QSP176 (SEQ ID NO: 56) is a peptide having the amino acid sequence GSLSTFFALFNRSFTQALGK (SEQ ID NO: 79; QSP176-A9) or the amino acid sequence GSLSTFFRLFNASFTQALGK (SEQ ID NO: 80;QSP176-A13).

**11.** Peptide antagonist according to claim 10, for use as a medicine.

**12.** Peptide antagonist of one or more of the peptides selected from the group consisting of: CVGIW (SEQ ID NO: 11; QSP022), ESRVSRIILDFLFQRKK (SEQ ID NO: 28; QSP055), and KSSAYSLQMGATAIKQVKKLFKKWGW (SEQ ID NO: 40; QSP0125), SGSLSTFFRLFNRSFTQALGK (SEQ ID NO: 56; QSP176), and SIFTLVA (SEQ ID NO: 57; QSP184), wherein the antagonist is a peptide having one or two deleted or substituted amino acids in said resp. peptide sequence, for use in preventing, reducing or treating muscle wasting associated with muscular dystrophies, amyotrophic lateral sclerosis, inflammatory myopathies, denervation muscle atrophies, neurological disease, cachexia, anorexia-cachexia syndrome, cancers, rheumatoid arthritis, osteoarthritis, diabetes, inflammatory bowel disease, liver cirrhosis, chronic obstructive pulmonary disease, pulmonary fibrosis, chronic renal disease, chronic heart failure, sarcopenia, physical frailty, androgen deprivation, corticosteroid myopathy, trauma (e.g. burns), critical illness myopathy (e.g. sepsis), acquired immune deficiency syndrome (AIDS) or cardiomyopathy.

**Patentansprüche**

**1.** In-vitro-Diagnoseverfahren zum Analysieren des Vorhandenseins eines Quorum-Sensormoleküls (QSM, Quorum sensing molecule) in einer Probe eines Säugers, um das Vorhandensein von mindestens einem QSM zu bestimmen, das Muskelschwund beeinflusst, und wobei das QSM ausgewählt wird aus der Gruppe bestehend aus: CVGIW (SEQ ID NO: 11; QSP022), ESRVSRIILDFLFQRKK (SEQ ID NO: 28; QSP055), KSSAYSLQMGATAIKQVKKLFKKWGW (SEQ ID NO: 40; QSP0125), SGSLSTFFRLFNRSFTQALGK (SEQ ID NO: 56; QSP176) und SIFTLVA (SEQ ID NO: 57; QSP184).

**2.** Diagnoseverfahren gemäß Anspruch 1, wobei das Verfahren auf Fäzes und/oder Blut und/oder Speichel und/oder Hautabstrichen und/oder anderen Körperflüssigkeiten und/oder anderen Proben des Säugers ausgeführt wird.

**3.** Diagnoseverfahren gemäß einem der Ansprüche 1-2, wobei der Säuger ein Mensch, ein Haustier oder ein Nutztier ist.

**4.** Diagnoseverfahren gemäß einem der Ansprüche 1-3, wobei das Vorhandensein von mindestens einem QSM auf ein Risiko oder das Vorhandensein von Muskelschwund hinweist.

**5.** Bakterienstamm der Spezies *Lactobacillus plantarum, Enterococcus faecalis, Streptococcus mitis* oder *Streptococcus mutans,* wobei der Stamm nicht eines oder mehrere der QSMs, ausgewählt aus der Gruppe bestehend aus: CVGIW (SEQ ID NO: 11; QSP022), ESRVSRIILDFLFQRKK (SEQ ID NO: 28; QSP055), KSSAYSLQMGATAIKQVKKLFKKWGW (SEQ ID NO: 40; QSP0125), SGSLSTFFRLFNRSFTQALGK (SEQ ID NO: 56; QSP176) und SIFTLVA (SEQ ID NO: 57; QSP184), produziert, zur Verwendung bei der Verhinderung, Behandlung oder Verringerung von Muskelschwund im Zusammenhang mit Muskeldystrophien, amyotropher Lateralsklerose, entzündlichen Myopathien, denervierenden Muskelatrophien, neurologischen Erkrankungen, Kachexie, Anorexie-Kachexie-Syndrom, Krebs, rheumatoider Arthritis, Osteoarthritis, Diabetes, entzündlicher Darmerkrankung, Leberzirrhose, chronisch obstruktiver Lungenerkrankung, Lungenfibrose, chronischer Nierenerkrankung, chronischer Herzinsuffizienz, Sarkopenie, körperlicher Gebrechlichkeit, Androgendeprivation, Kortikosteroidmyopathie, Trauma (z. B. Verbrennungen), Myopathie bei kritischer Erkrankung (z. B. Sepsis), erworbenem Immunschwächesyndrom (AIDS) oder Kardiomyopathie.

6. Bakterienstamm zur Verwendung gemäß Anspruch 5, wobei der Stamm die Stämme, die QSMs aufweisen, die nachweislich Aspekte der Muskelhomöostase negativ beeinflussen, im Darm ersetzt oder verringert.

7. Bakterienstamm zur Verwendung gemäß Anspruch 6, wobei die Stämme, die QSMs aufweisen, die nachweislich Aspekte der Muskelhomöostase negativ beeinflussen, Bakterienstämme sind, die ein oder mehrere der QSMs, ausgewählt aus der Gruppe bestehend aus: CVGIW (SEQ ID NO: 11; QSP022), ESRVSRIILDFLFQRKK (SEQ ID NO: 28; QSP055), KSSAYSLQMGATAIKQVKKLFKKWGW (SEQ ID NO: 40; QSP0125), SGSLSTFFRLFNRSFTQALGK (SEQ ID NO: 56; QSP176) und SIFTLVA (SEQ ID NO: 57; QSP184), produzieren.

8. Bakterienstamm zur Verwendung gemäß einem der Ansprüche 5-7, wobei der Bakterienstamm Teil einer Zusammensetzung, insbesondere eines lebenden biotherapeutischen Produkts oder Probiotikums, ist.

9. Verfahren zum Screening von als lebendes biotherapeutisches Produkt oder probiotischer Stamm geeigneten Bakterienstämmen, wobei das von dem Bakterienstamm produzierte QSM analysiert wird, und wobei das Nichtvorhandensein von einem oder mehreren der QSMs, ausgewählt aus der Gruppe bestehend aus: CVGIW (SEQ ID NO: 11; QSP022), ESRVSRIILDFLFQRKK (SEQ ID NO: 28; QSP055), KSSAYSLQMGATAIKQVKKLFKKWGW (SEQ ID NO: 40; QSP0125), SGSLSTFFRLFNRSFTQALGK (SEQ ID NO: 56; QSP176) und SIFTLVA (SEQ ID NO: 57; QSP184), ein Hinweis auf einen Stamm ist, der als lebendes biotherapeutisches Produkt oder Probiotikum geeignet ist.

10. Peptidantagonist von einem oder mehreren der Peptide, ausgewählt aus der Gruppe bestehend aus: ESRVSRIILDFLFQRKK (SEQ ID NO: 28; QSP055) und SGSLSTFFRLFNRSFTQALGK (SEQ ID NO: 56; QSP176), wobei der Peptidantagonist von QSP055 (SEQ ID NO: 28) ein Peptid mit der Aminosäuresequenz SRVSAIILDFLFQRKK (SEQ ID NO: 77; QSP055-A6) oder der Aminosäuresequenz ESRVSRIIADFLFQRKK (SEQ ID NO: 78; QSP055-A9) ist, und wobei der Peptidantagonist von QSP176 (SEQ ID NO: 56) ein Peptid mit der Aminosäuresequenz GSLSTFFALFNRSFTQALGK (SEQ ID NO: 79; QSP176-A9) oder der Aminosäuresequenz GSLSTFFRLFNASFTQALGK (SEQ ID NO: 80; QSP176-A13) ist.

11. Peptidantagonist gemäß Anspruch 10 zur Verwendung als Medikament.

12. Peptidantagonist von einem oder mehreren der Peptide, ausgewählt aus der Gruppe bestehend aus: CVGIW (SEQ ID NO: 11; QSP022), ESRVSRIILDFLFQRKK (SEQ ID NO: 28; QSP055) und KSSAYSLQMGATAIKQVKKLFKKWGW (SEQ ID NO: 40; QSP0125), SGSLSTFFRLFNRSFTQALGK (SEQ ID NO: 56; QSP176) und SIFTLVA (SEQ ID NO: 57; QSP184), wobei der Antagonist ein Peptid, aufweisend eine oder zwei deletierte oder substituierte Aminosäuren in der jeweiligen Peptidsequenz, ist, zur Verwendung bei der Verhinderung, Verringerung oder Behandlung von Muskelschwund im Zusammenhang mit Muskeldystrophien, amyotropher Lateralsklerose, entzündlichen Myopathien, denervierenden Muskelatrophien, neurologischen Erkrankungen, Kachexie, Anorexie-Kachexie-Syndrom, Krebs, rheumatoider Arthritis, Osteoarthritis, Diabetes, entzündlicher Darmerkrankung, Leberzirrhose, chronisch obstruktiver Lungenerkrankung, Lungenfibrose, chronischer Nierenerkrankung, chronischer Herzinsuffizienz, Sarkopenie, körperlicher Gebrechlichkeit, Androgendeprivation, Kortikosteroidmyopathie, Trauma (z. B. Verbrennungen), Myopathie bei kritischer Erkrankung (z. B. Sepsis), erworbenem Immunschwächesyndrom (AIDS) oder Kardiomyopathie.

**Revendications**

1. Procédé *in vitro* de diagnostic pour l'analyse de la présence d'une molécule de détection de quorum (QSM) dans un échantillon d'un mammifère pour évaluer la présence d'au moins une QSM qui influence la fonte musculaire et la QSM étant choisie dans le groupe constitué par :
CVGIW (SEQ ID NO: 11; QSP022), ESRVSRIILDFLFQRKK (SEQ ID NO: 28; QSP055), KSSAYSLQMGATAIKQVKKLFKKWGW (SEQ ID NO: 40; QSP0125), SGSLSTFFRLFNRSFTQALGK (SEQ ID NO: 56; QSP176), et SIFTLVA (SEQ ID NO: 57; QSP184).

2. Procédé de diagnostic selon la revendication 1, le procédé étant réalisé sur des selles et/ou du sang et/ou de la salive et/ou des écouvillons de peau et/ou d'autres fluides corporels et/ou d'autres échantillons du mammifère.

3. Procédé de diagnostic selon l'une quelconque des revendications 1 à 2, le mammifère étant un humain, un animal de compagnie ou un animal de ferme.

4. Procédé de diagnostic selon l'une quelconque des revendications 1 à 3, la présence d'au moins une QSM indiquant un risque de ou la présence d'une fonte musculaire.

5. Souche bactérienne de l'espèce *Lactobacillus* plantarum, *Enterococcus faecalis, Streptococcus mitis* ou *Streptococcus mutans*, ladite souche ne produisant pas une ou plusieurs des QSM choisies dans le groupe constitué CVGIW (SEQ ID NO: 11; QSP022),ESRVSRIILDFLFQRKK (SEQ ID NO: 28; QSP055), KSSAYSLQMGATAIKQVKKLFKKWGW (SEQ ID NO 40. QSP0125), SGSLSTFFRLFNRSFTQALGK (SEQ ID NO 56; QSP176), et SIFTLVA (SEQ ID NO: 57; QSP184)
par : ,
pour une utilisation dans la prévention, le traitement ou la réduction d'une fonte musculaire associée aux dystrophies musculaires, à la sclérose latérale amyotrophique, aux myopathies inflammatoires, aux atrophies musculaires de dénervation, aux maladies neurologiques, à la cachexie, au syndrome d'anorexie-cachexie, aux cancers, à la polyarthrite rhumatoïde, à l'arthrose, au diabète, aux maladies inflammatoires de l'intestin, à la cirrhose du foie, à la bronchopneumopathie chronique obstructive, à la fibrose pulmonaire, à la maladie rénale chronique, à l'insuffisance cardiaque chronique, à la sarcopénie, à la fragilité physique, à la privation d'androgènes, à la myopathie aux corticostéroïdes, à un traumatisme (par exemple, des brûlures), à une myopathie liée à une maladie grave (par exemple, une septicémie), au syndrome d'immunodéficience acquise (SIDA) ou à une cardiomyopathie.

6. Souche bactérienne pour une utilisation selon la revendication 5, ladite souche remplaçant ou réduisant dans l'intestin les souches qui possèdent des QSM montrées comme influençant négativement des aspects de l'homéostasie musculaire.

7. Souche bactérienne pour une utilisation selon la revendication 6, les souches qui possèdent des QSM montrées comme influençant négativement des aspects de l'homéostasie musculaire étant des souches bactériennes qui produisent une ou plusieurs des QSM choisies dans le groupe constitué par :
CVGIW (SEQ ID NO: 11; QSP022), ESRVSRIILDFLFQRKK (SEQ ID NO: 28; QSP055), KSSAYSLQMGATAIKQVKKLFKKWGW (SEQ ID NO: 40; QSP0125), SGSLSTFFRLFNRSFTQALGK (SEQ ID NO: 56; QSP176), et SIFTLVA (SEQ ID NO: 57, QSP184).

8. Souche bactérienne pour une utilisation selon l'une quelconque des revendications 5 à 7, la souche bactérienne faisant partie d'une composition, en particulier d'un produit biothérapeutique vivant ou d'un probiotique.

9. Procédé de criblage de souches bactériennes appropriées comme produit biothérapeutique vivant ou souche probiotique, la QSM produite par ladite souche bactérienne étant analysée, et l'absence d'une ou plusieurs des QSM choisies dans le groupe constitué par
CVGIW (SEQ ID NO: 11, QSP022), ESRVSRIILDFLFQRKK (SEQ ID NO: 28, QSP055), KSSAYSLQMGATAIKQVKKLFKKWGW (SEQ ID NO: 40. QSP0125). SGSLSTFFRLFNRSFTQALGK (SEQ ID NO 56; QSP176), et SIFTLVA (SEQ ID NO: 57; QSP184)
indiquant une souche qui est appropriée comme produit biothérapeutique vivant ou un probiotique.

10. Antagoniste peptidique parmi l'un ou plusieurs parmi les peptides choisis dans le groupe constitué par : ESRVSRIILDFLFQRKK (SEQ ID NO: 28; QSP055),. et SGSLSTFFRLFNRSFTQALGK (SEQ ID NO: 56; QSP176), l'antagoniste peptidique de QSP055 (SEQ ID NO: 28) étant un peptide ayant la séquence d'acides aminés SRVSAIILDFLFQRKK (SEQ ID NO: 77; QSP055-A6) ou la séquence d'acides aminés ESRVSRIIADFLFQRKK (SEQ ID NO: 78; QSP055-A9), et l'antagoniste peptidique de QSP176 (SEQ ID NO: 56) étant un peptide ayant la séquence d'acides aminés GSLSTFFALFNRSFTQALGK (SEQ ID NO: 79; QSP176-A9) ou la séquence d'acides aminés GSLSTFFRLFNASFTQALGK (SEQ ID NO: 80;QSP176-A13).

**11.** Antagoniste peptidique selon la revendication 10, pour une utilisation comme un médicament.

**12.** Antagoniste peptidique parmi l'un ou plusieurs parmi les peptides choisis dans le groupe constitué par :
CVGIW (SEQ ID NO: 11; QSP022), ESRVSRIILDFLFQRKK (SEQ ID NO: 28; QSP055), KSSAYSLQMGATAIKQVKKLFKKWGW (SEQ ID NO: 40; QSP0125), SGSLSTFFRLFNRSFTQALGK (SEQ ID NO: 56; QSP176), et SIFTLVA (SEQ ID NO: 57; QSP184),
l'antagoniste étant un peptide ayant un ou deux acides aminés délétés ou substitués dans ladite séquence peptidique respective, pour une utilisation dans la prévention, la réduction ou le traitement d'une fonte musculaire associée aux dystrophies musculaires, à la sclérose latérale amyotrophique, aux myopathies inflammatoires, aux atrophies musculaires de dénervation, aux maladies neurologiques, à la cachexie, au syndrome d'anorexie-cachexie, aux cancers, à la polyarthrite rhumatoïde, à l'arthrose, au diabète, aux maladies inflammatoires de l'intestin, à la cirrhose du foie, à la bronchopneumopathie chronique obstructive, à la fibrose pulmonaire, à la maladie rénale chronique, à l'insuffisance cardiaque chronique, à la sarcopénie, à la fragilité physique, à la privation d'androgènes, à la myopathie aux corticostéroïdes, à un traumatisme (par exemple, des brûlures), à une myopathie liée à une maladie grave (par exemple, une septicémie), au syndrome d'immunodéficience acquise (SIDA) ou à une cardiomyopathie.

Figure 1

Figure 2

Figure 3

Figure 4

A

B

Figure 4

C

D

Figure 5

Primary hits

EP 4 055 389 B1

Figure 6A

**IL-6**

Figure 6B

**IL-6**

Figure 7

MTT reduction

Figure 8A

IL-6 Q055 + Ala scans

Figure 8B

MTT Q176 + Ala scans

Figure 8C

IL-6 Q176 + Ala scans

Figure 9

Figure 10

Figure 10 – continued

**e**

**f**

**g**

**h**

Figure 11

Figure 11 – continued

Figure 12

Q055 (human paravertebral myotubes)

Q125 (human quadriceps myotubes)

Q176 (human quadriceps myotubes)

Figure 13

A

## Q055

B

## Q125

C

## Q176

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20150335688 A1

### Non-patent literature cited in the description

- **E. WYNENDAELE ; F. VERBEKE ; M. D'HONDT ; A. HENDRIX ; C. VAN DE WIELE ; C. BURVENICH ; K. PEREMANS ; O. DE WEVER ; M. BRACKE ; B. DE SPIEGELEER.** Crosstalk between the microbiome and cancer cells by quorum sensing peptides. *Peptides,* 2015, vol. 64, 40-48
- **B. DE SPIEGELEER ; F. VERBEKE ; M. D'HONDT ; A. HENDRIX ; C. VAN DE WIELE ; C, BURVENICH ; K. PEREMANS ; O. DE WEVER ; M. BRACKE ; E. WYNENDAELE.** The Quorum Sensing Peptides PhrG, CSP and EDF Promote Angiogenesis and Invasion of Breast Cancer Cells In Vitro. *Plos One,* 2015, vol. 10 (3
- **Y. JANSSENS ; E. WYNENDAELE ; F. VERBEKE ; N. DEBUNNE ; B. GEVAERT ; K. AUDENAERT ; C. VAN DE WIELE ; B. DE SPIEGELEER.** Screening of quorum sensing peptides for biological effects in neuronal cells. *Peptides,* 2018, vol. 101, 150-156
- **A. BANDYOPADHAYA ; C. CONSTANTINOU ; N. PSYCHOGIOS ; R. UEKI ; S. YASUHARA ; J. MARTYN ; J. WILHELMY ; M. MINDRINOS ; L.G. RAHME ; A.A. TZIKA.** Bacterial-excreted small volatile molecule 2-aminoacetophenone induces oxidative stress and apoptosis in murine skeletal muscle. *International Journal of Molecular Medicine,* 2016, vol. 37, 867-878
- **B. DE SPIEGELEER ; F. VERBEKE ; M. D'HONDT ; A. HENDRIX ; C. VAN DE WIELE ; C. BURVENICH ; K. PEREMANS ; O. DE WEVER ; M. BRACKE ; E. WYNENDAELE.** The Quorum Sensing Peptides PhrG, CSP and EDF Promote Angiogenesis and Invasion of Breast Cancer Cells In Vitro. *Plos One,* 2015, vol. 10 (3
- **Y. JANSSENS ; E. WYNENDAELE ; F. VERBEKE ; N. DEBUNNE ; B. GEVAERT ; K. AUDENAERT ; C. VAN DE WIELE ; B. DE SPIEGELEER.** Screening of quorum sensing peptides for biological effects in neuronal cells. *Peptides,* 2018, vol. 101, 150-156
- **F. VERBEKE et al.** Peptides as quorum sensing molecules: measurement techniques and obtained levels in vitro and in vivo. *Front. Neurosci.,* 2017, vol. 11, 183
- **F. VERBEKE et al.** *Journal of Pharmaceutical and Biomedical Analysis,* 2018, vol. 160, 55-63
- **N. DEBUNNE et al.** *Chromatographia,* 2018, vol. 81, 25-40
- **C. BRANDT et al.** *Journal of Biomedicine and Biotechnology,* 2010
- **S. WELC et al.** *Exp. Physiol.,* 2013, vol. 98 (2), 359-371
- **R. MCCOLL ; M. NKOSI ; C. SNYMAN ; C. NIESLER.** Analysis and quantification of in vitro myoblast fusion using the LADD Multiple Stain. *Biotechniques,* 2016, vol. 61 (6), 323-326
- **X.H.D. ZHANG.** Illustration of SSMD, z Score, SSMD*, z* Score, and t Statistic for Hit Selection in RNAi High-Throughput Screens. *Journal of Biomolecular Screening,* 2011, vol. 16 (7), 775-785
- **T. VALIKANGAS ; T. SUOMI ; L.L. ELO.** A systematic evaluation of normalization methods in quantitative label-free proteomics. *Briefings in Bioinformatics,* 2018, vol. 19 (1), 1-11
- **X.H.D. ZHANG ; X.T.C. YANG ; N.J. CHUNG ; A. GATES ; E. STEC ; P. KUNAPULI ; D.J. HOLDER ; M. FERRER ; A.S. ESPESETH.** Robust statistical methods for hit selection in RNA interference high-throughput screening experiments. *Pharmacogenomics,* 2006, vol. 7 (3), 299-309
- **E. WYNENDAELE ; B. GEVAERT ; S. STALMANS ; F. VERBEKE ; B. DE SPIEGELEER.** Exploring the Chemical Space of Quorum Sensing Peptides. *Biopolymers,* 2015, vol. 104 (5), 544-551
- **RESTIF et al.** *Plos Computational Biology,* 2014
- **F. VERBEKE ; S. DE CRAEMER ; N. DEBUNNE ; Y. JANSSENS ; E. WYNENDAELE ; C. VAN DE WIELE ; B. DE SPIEGELEER.** Peptides as Quorum Sensing Molecules: Measurement Techniques and Obtained Levels In vitro and In vivo. *Frontiers in Neuroscience,* 2017, vol. 11, 1-18